(19) Europäisches Patentamt
European Patent Office
Office européen des brevets

(11) **EP 4 428 864 A1**

(12) **EUROPEAN PATENT APPLICATION**
published in accordance with Art. 153(4) EPC

(43) Date of publication:
**11.09.2024 Bulletin 2024/37**

(21) Application number: **22890317.5**

(22) Date of filing: **01.11.2022**

(51) International Patent Classification (IPC):
*G16B 35/00* (2019.01)    *G16B 30/10* (2019.01)
*G16B 5/00* (2019.01)    *G16B 40/20* (2019.01)
*C12Q 1/6886* (2018.01)    *C12Q 1/6806* (2018.01)

(52) Cooperative Patent Classification (CPC):
**C12Q 1/6806; C12Q 1/6886; G16B 5/00; G16B 30/10; G16B 35/00; G16B 40/20**

(86) International application number:
**PCT/KR2022/016868**

(87) International publication number:
**WO 2023/080586 (11.05.2023 Gazette 2023/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.11.2021 KR 20210149466**

(71) Applicant: **GC Genome Corporation**
**Yongin-si, Gyeonggi-do 16924 (KR)**

(72) Inventors:
• **CHO, EunHae**
**Yongin-si Gyeonggi-do 16924 (KR)**
• **LEE, Tae-Rim**
**Yongin-si Gyeonggi-do 16924 (KR)**

(74) Representative: **Mewburn Ellis LLP**
**Aurora Building**
**Counterslip**
**Bristol BS1 6BX (GB)**

(54) **METHOD FOR DIAGNOSING CANCER BY USING SEQUENCE FREQUENCY AND SIZE AT EACH POSITION OF CELL-FREE NUCLEIC ACID FRAGMENT**

(57)    The present invention relates to a method for diagnosing cancer and predicting cancer type by using the terminal sequence frequency and the size of a cell-free nucleic acid fragment, and, more specifically, to a method for diagnosing cancer and predicting cancer type by using a method for extracting nucleic acids from a biospecimen so as to derive the terminal sequence frequency of a nucleic acid fragment and the size of the nucleic acid fragment on the basis of a read obtained by acquiring and aligning sequence information, generating vectorized data from same, and then inputting the data into a trained artificial intelligence model so as to analyze a calculated value. The method for diagnosing cancer and predicting cancer type by using the terminal sequence frequency and the size of a cell-free nucleic acid fragment, according to the present invention, generates vectorized data and analyzes same by using an AI algorithm, thereby exhibiting high sensitivity and accuracy even if read coverage is low, and thus is useful.

FIG. 1

Extracting cell-free nucleic acids from plasma

Acquiring nucleic acid fragments by using massively parallel sequencing method

Aligning nucleic acid fragment data human reference chromosomes and Determining quality of aligned data

Measuring length of aligned nucleic acid fragments

Calculating nucleic acid sequence frequency at each position of nucleic acid fragment in nucleic acid fragment group with same size

Screening the optimal combinations of nucleic acid fragment size, position and base sequence to distinguish between healthy individuals and cancer patients

Training machine learning model

Calculating probability index

Determining whether the subject is a cancer patient

EP 4 428 864 A1

## Description

### Technical Field

The present invention relates to a method for diagnosing cancer by using sequence relative frequency and size at each position of a cell-free nucleic fragment, and, more particularly, to a method for diagnosing cancer using a method for extracting nucleic acids from a biospecimen so as to derive the sequence relative frequency and the size at each position of the nucleic acid fragment on the basis of a read obtained by acquiring and aligning sequence information, and then inputting the data into a trained artificial intelligence model so as to analyze a calculated value.

### Related Art

The diagnosis of cancer in clinical practice is usually confirmed by case history study, physical examination, and clinical evaluation, followed by a tissue biopsy. A clinical diagnosis of cancer is only possible when the number of cancer cells is more than one billion and the diameter of the cancer is more than one centimeter. In this case, the cancer cells already have the ability to metastasize, and at least half of them have already metastasized. In addition, biopsies are invasive, causing considerable discomfort to the patient, and it is often not possible to perform a biopsy while treating a cancer patient. In addition, tumor markers are used in cancer screening to monitor substances produced directly or indirectly by cancer, but their accuracy is limited because more than half of tumor marker screening results are normal even in the presence of cancer, and they are often positive even in the absence of cancer.

In response to the need for a relatively simple, non-invasive, and highly sensitive and specific cancer diagnostic method that can compensate for the shortcomings of conventional cancer diagnostic methods, liquid biopsy, which utilizes a patient's body fluids to diagnose and follow up on cancer, has recently been widely used. Liquid biopsy is a non-invasive method that is drawing attention as an alternative to existing invasive diagnostic and testing methods.

Recently, methods for performing cancer diagnosis and cancer type differentiation by using cell free DNA obtained from liquid biopsy have been developed (US 10975431, Zhou, Xionghui et al., bioRxiv, 2020.07.16.201350), and in particular, methods for analyzing motif frequency information of cell free nucleic acid terminal sequence for use in cancer diagnosis, prenatal diagnosis, or organ transplant monitoring are known (WO 2020-125709, Peiyong Jiang et al., cancer discovery, Vol. 10, 2020, pp. 664-673).

On the other hand, the Gradient Boosting Algorithm (GBM) is a predictive model that can perform regression or classification analysis and belongs to the boosting family of ensemble methodologies. The Gradient Boosting Algorithm shows tremendous performance in predicting tabular data (data in an X-Y grid such as Excel) and is known to have the highest prediction performance among machine learning algorithms.

Although there are a variety of literatures (Daping Yu et al., Thoracic Cancer Vol. 11, pp. 95-102. 2020, KR 10-2061800, KR 10- 2108050, KR 10-2021-0081547) using this Gradient Boosting Algorithm for use in the bio field, there is a lack of research on how to diagnose cancer through GBM based on sequencing information of cell-free DNA (cfDNA) in blood.

Accordingly, the inventors of the present invention have made good faith efforts to solve the above problems and develop an artificial intelligence-based cancer diagnosis method with high sensitivity and accuracy, and have confirmed that if the optimal sequence relative frequency and size combination are selected based on the sequence relative frequency at each position of a cell-free nucleic acid fragment and the size information of nucleic acid fragment, and analyzed by a trained artificial intelligence model, cancer diagnosis can be performed with high sensitivity and accuracy, and have completed the present invention.

### Summary of Invention

An object of the present invention is to provide a method for diagnosing cancer by using the sequence relative frequency and the size at each position of a cell-free nucleic fragment.

Another object of the present invention is to provide a cancer diagnostic device utilizing the sequence relative frequency and the size at each position of a cell-free nucleic fragment.

Another object of the present invention is to provide a computer-readable storage medium comprising instructions configured to be executed by a processor performing a cancer diagnosis in the above manner.

To accomplish the above objectives, the present invention provides a method of providing information for diagnosing cancer using a cell-free nucleic acid, comprising the steps of (a) obtaining a sequence information from extracted nucleic acid from a biological sample; (b) aligning the obtained sequence information (reads) to a reference genome database; (c) deriving the sequence relative frequency and the size at each position of nucleic acid fragments using the aligned sequence information (reads); and (d) inputting the derived sequence relative frequency and size information to an artificial intelligence model trained to diagnose cancer and comparing the analyzed output with a cut-off value to determine the presence or absence of cancer, wherein the artificial intelligence model is trained to distinguish normal samples and cancer samples based on the sequence relative frequency at each position of the nucleic acid fragment and the size information of the nucleic acid fragment.

The present invention also provides a cancer diagnostic device comprising: a decoding part that extracts nucleic acid from a biospecimen and decodes sequence information; an alignment part that aligns the decoded sequence to a reference genome database; a nucleic acid fragment analysis part that derives the sequence relative frequency at each position of the nucleic acid fragment and the size of the nucleic acid fragment based on the aligned sequence; and a cancer diagnostic part that inputs the derived sequence relative frequency at each position of the nucleic acid fragment and size information of the nucleic acid fragment into a trained artificial intelligence model, analyzes same, and determines the presence of cancer by comparing the analyzed output to a cut-off value.

The invention also relates to a computer-readable storage medium comprising instructions configured to be executed by a processor to provide information for diagnosing cancer, the instructions comprising (a) obtaining a sequence information from extracted nucleic acid from a biological sample; (b) aligning the obtained sequence information (reads) to a reference genome database; (c) deriving the sequence relative frequency and the size at each position of nucleic acid fragments using the aligned sequence information (reads); and (d) inputting the derived sequence relative frequency and size information to an artificial intelligence model trained to diagnose cancer and comparing the analyzed output with a cut-off value to determine the presence or absence of cancer, wherein the artificial intelligence model is trained to distinguish normal samples and cancer samples based on the sequence relative frequency at each position of the nucleic acid fragment and the size information of the nucleic acid fragment.

The present invention also provides a method for diagnosing cancer using a cell-free nucleic acid, comprising the steps of (a) obtaining a sequence information from extracted nucleic acid from a biological sample; (b) aligning the obtained sequence information (reads) to a reference genome database; (c) deriving the sequence relative frequency and the size at each position of nucleic acid fragments using the aligned sequence information (reads); and (d) inputting the derived sequence relative frequency and size information to an artificial intelligence model trained to diagnose cancer and comparing the analyzed output with a cut-off value to determine the presence or absence of cancer, wherein the artificial intelligence model is trained to distinguish normal samples and cancer samples based on the sequence relative frequency at each position of the nucleic acid fragment and the size information of the nucleic acid fragment.

**Brief Description of Drawing**

FIG. 1 is an overall flowchart for performing the cancer diagnostic method of the present invention using the sequence relative frequency and the size at each position of a cell-free nucleic acid fragment.

FIG. 2 is an example of a process for screening the nucleic acid fragment sizes that have statistically significant differences in relative frequency by size between healthy individuals and cancer patients in one embodiment of the present invention.

FIG. 3 is a graph showing statistical values of the relative frequency of the nucleic acid fragments by size identified in one embodiment of the present invention and a size distribution of the selected nucleic acid fragments.

FIG. 4 is a heatmap visualization of a FESS table produced by one embodiment of the present invention.

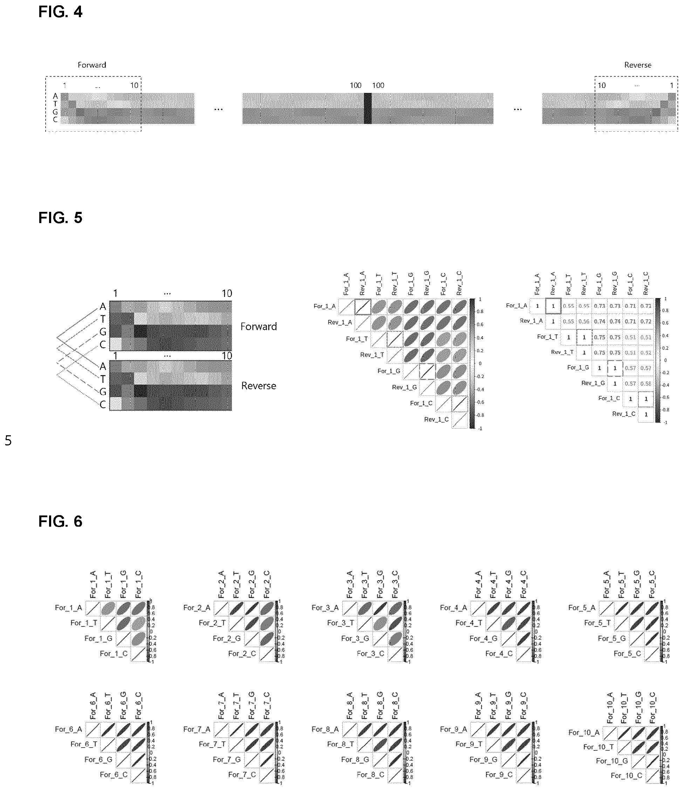

The left panel of FIG. 5 shows a zoomed-in view of the dotted portion in FIG. 4, and the right two panels show a statistical analysis of the relative frequency of base sequences at each position.

FIG. 6 shows a statistical confirmation of the similarity between the base sequences of A, T, G and C by calculating the relative frequency of each sequence at the location of the nucleic acid fragments selected in one embodiment of the present invention.

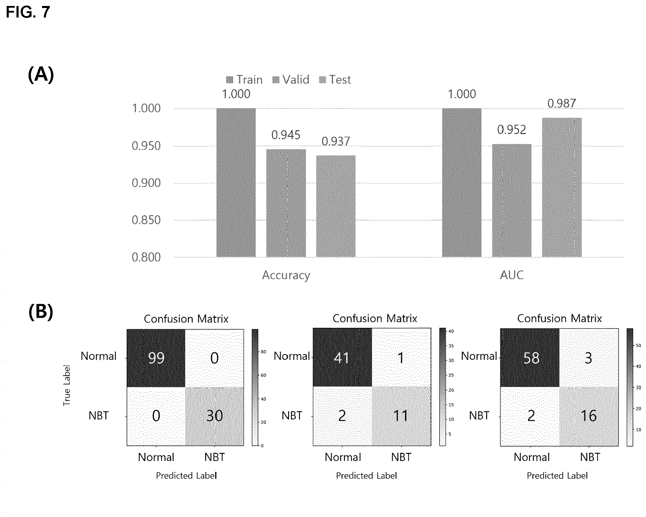

In FIG. 7, (A) is the performance of the machine learning model built in one embodiment of the present invention as measured by Accuracy and AUC, and (B) is the confusion matrix.

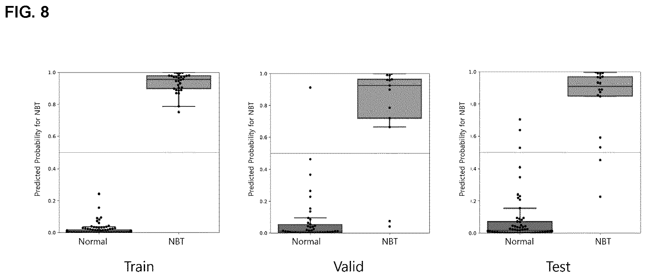

FIG. 8 shows how well the probability values of healthy individuals and neuroblastoma patients predicted by the machine learning model built in one embodiment of the present invention match the actual patients, as determined by the distribution of XPI values output by the machine learning model.

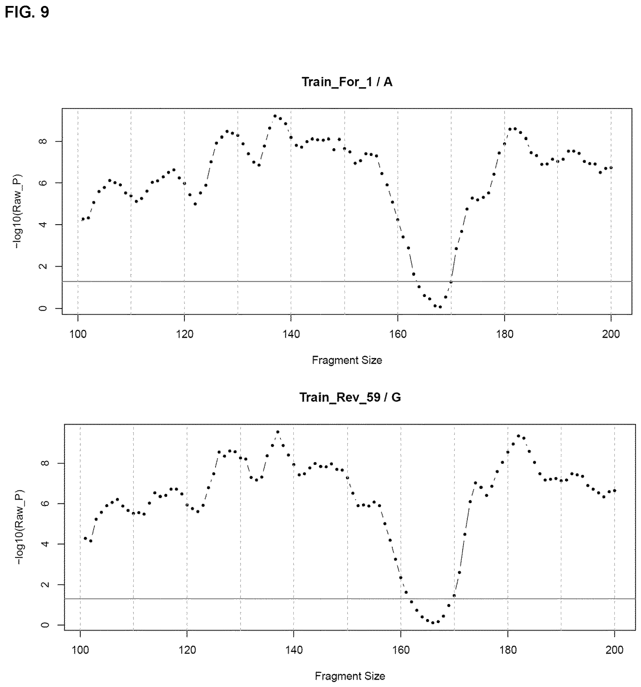

FIG. 9 is a graph showing statistical values of the relative frequency of nucleic acid fragments by size identified in one embodiment of the present invention and the size distribution of the selected nucleic acid fragments at different positions and bases.

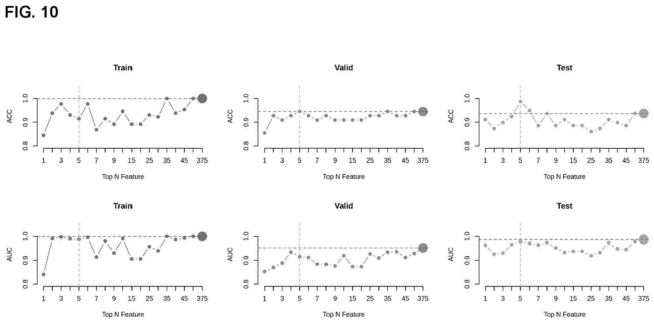

FIG. 10 shows the performance of a machine learning model built with a small number of features according to the importance of the selected features in one embodiment of the present invention, where the top panel is Accuracy and the bottom panel is Area Under Curve (AUC).

**Detailed Description of the invention and Preferred Embodiments**

Unless otherwise defined, all technical and scientific terms used herein have the same meaning as commonly understood by a person skilled in the art. In general, the nomenclature used herein is well known and in common use in the art.

The terms "first," "second," "A," "B," and the like may be used to describe various components, but the components are not limited by such terms and are used only to distinguish one component from another. For example, without departing from the scope of the technology described herein, a "first component" may be named a "second component," and similarly, a "second component" may be named a "first component." The term "and/or" includes any combination of a plurality of related recited components or any of a plurality of related recited components.

As used herein, the singular expression shall be understood to include the plural expression unless the context clearly indicates otherwise, and the term "comprising" shall be understood to mean the presence of the features, numbers, steps, actions, components, parts, or combinations thereof set forth, and not to exclude the possibility of the presence or addition of one or more other features, numbers, steps, actions components, parts, or combinations thereof.

Before proceeding to a detailed description of the drawings, it should be clarified that the division of the components herein is only by the primary function performed by each component, i.e., two or more of the components described herein may be combined into a single component, or a single component may be divided into two or more components with more detailed functions. Each of the components described herein may additionally perform some or all of the functions of other components in addition to their own primary functions, and some of the primary functions of each component may be dedicated and performed by other components.

Further, in performing the method or operation method, the steps comprised in the method may occur in a different order from that specified unless the context clearly indicates a particular order, i.e., the steps may occur in the same order as specified, may be performed substantially simultaneously, or may be performed in reverse order.

In the present invention, it was sought to confirm that cancer diagnosis can be performed with high sensitivity and accuracy by aligning sequence analysis data obtained from a sample to a reference genome, then deriving the sequence relative frequency at each position of the nucleic acid fragment and the size of the nucleic acid fragment based on the aligned sequence information, inputting the above derived sequence relative frequency at each position of the nucleic acid fragment and size of the nucleic acid fragment into a trained artificial intelligence model, and then calculating and analyzing the XPI value.

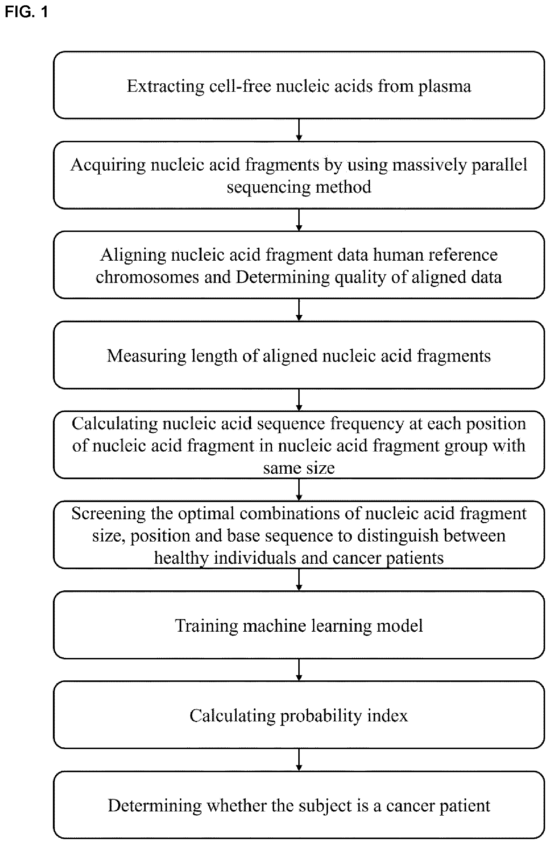

In other words, in one embodiment of the present invention, a method for performing cancer diagnosis is developed by sequencing DNA extracted from blood, aligning it to a reference chromosome, using same to derive the sequence relative frequency at each position of the nucleic acid fragment and the size of nucleic acid fragment, deriving an optimal combination of sequence relative frequency at each position of nucleic acid fragment and size of nucleic acid fragment, training same with a deep learning model to calculate an XPI value, and comparing the analyzed output to a cut-off value (FIG. 1).

Accordingly, in one aspect, the present invention provides a method of providing information for diagnosing cancer using a cell-free nucleic acid, comprising the following steps:

(a) obtaining a sequence information from extracted nucleic acid from a biological sample;
(b) aligning the obtained sequence information (reads) to a reference genome database;
(c) using the aligned sequence information (reads) to derive the sequence relative frequency at each position of the nucleic acid fragment and the size of nucleic acid fragment; and
(d) inputting the derived sequence relative frequency and size information into an artificial intelligence model trained to diagnose cancer, and comparing the analyzed output to a cut-off value to determine the presence or absence of cancer,

wherein the artificial intelligence model is trained to distinguish normal samples and cancer samples based on the sequence relative frequency at each position of the nucleic acid fragment and the size information of the nucleic acid fragment.

In the present invention, the nucleic acid fragment can be any fragment of nucleic acid extracted from a biospecimen, preferably, a fragment of cell-free nucleic acid or intracellular nucleic acid, but is not limited thereto.

In the present invention, the nucleic acid fragment can be obtained by any method known to a person of ordinary skill in the art, preferably by direct sequencing, by sequencing by next-generation sequencing, non-specific whole genome amplification, or probe-based sequencing, but is not limited thereto.

In the present invention, the cancer may be a solid or blood cancer, preferably may be selected from the group consisting of non-Hodgkin lymphoma, non-Hodgkin lymphoma, acute-myeloid leukemia, acute-lymphoid leukemia, multiple myeloma, head and neck cancer, lung cancer, glioblastoma, colon/rectal cancer, pancreatic cancer, breast cancer, ovarian cancer, melanoma, prostate cancer, liver cancer, thyroid cancer, stomach cancer, gallbladder cancer, biliary tract cancer, bladder cancer, small intestine cancer, cervical cancer, cancer of unknown primary site, kidney cancer, esophageal cancer, neuroblastoma and mesothelioma, and more preferably, neuroblastoma, but is not limited thereto.

In the present invention, the step (a) comprises the steps of:

(a-i) obtaining nucleic acids from a biospecimen;
(a-ii) removing proteins, fats, and other residues from the collected nucleic acid by using a salting-out method, a column chromatography method, or a beads method to obtain purified nucleic acid;
(a-iii) creating a single-end sequencing or pair-end sequencing library from purified nucleic acids or nucleic acids that have been randomly fragmented by enzymatic cleavage, grinding, or hydroshear methods;
(a-iv) reacting the created library to a next-generation sequencer; and
(a-v) acquiring nucleic acid sequence information (reads) from a next-generation sequencer.

In the present invention, the step (a) of obtaining the sequence information may be characterized by, but is not limited to, obtaining the isolated cell-free DNA by wholegenome sequencing to a depth of 1 million to 100 million reads.

In the present invention, a biospecimen means any substance, biological fluid, tissue or cell obtained from or derived from an individual, for example, may include whole blood, leukocytes, peripheral blood mononuclear cells, leukocyte buffy coat, blood (including plasma and serum), sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, hair, oral cells, placental cells, cerebrospinal fluid, or mixtures thereof, but is not limited thereto.

As used in the present invention, the term "reference group" refers to a group of individuals who are currently free of a particular disease or condition, which is a reference group to which a comparison can be made, such as a standard sequence database. In the present invention, in a reference genome database of a reference group, the standard sequence may be a reference chromosome registered with a public health organization such as NCBI.

In the present invention, the nucleic acid in step (a) may be cell-free DNA, more preferably, circulating tumor cell DNA, but is not limited thereto.

In the present invention, the next-generation sequencer may be used with any sequencing method known in the art. Sequencing of the nucleic acids isolated by the selected method is typically performed by using next-generation sequencing (NGS). Next-generation sequencing includes any sequencing method that determines one nucleic acid sequence of an individual nucleic acid molecule or a clonally extended proxy for an individual nucleic acid molecule in a highly similar manner (e.g., 105 or more molecules are sequenced simultaneously). In one embodiment of the present invention, the relative abundance of a nucleic acid type in the library can be estimated by counting the relative number of occurrences of its homologous sequences in the data generated by the sequencing experiment. Next-generation sequencing methods are known in the art and are described, for example, in Metzker, M. (2010) Nature Biotechnology Reviews 11:31-46, which is incorporated herein by reference.

In one embodiment of the present invention, next-generation sequencing is used to determine the nucleic acid sequence of individual nucleic acid molecules (e.g., HeliScope Gene Sequencing system from Helicos BioSciences and PacBio RS system from Pacific Biosciences). In other embodiments, sequencing, for example, massively parallel, short-read sequencing that produces more bases of sequence per unit of sequencing than other sequencing methods that produce fewer but longer reads (e.g., Solexa sequencer from Illumina Inc. located in San Diego, CA) determines the nucleic acid sequence of a clonally extended proxy for an individual nucleic acid molecule (e.g., Solexa sequencer from Illumina Inc. located in San Diego, CA; 454 Life Sciences (Branford, CT) and Ion Torrent). Other methods or machines for next-generation sequencing are provided by, but not limited to, 454 Life Sciences (Branford, CT), Applied Biosystems (Foster City, CA; SOLiD sequencer), Helikos Bioscience Corporation (Cambridge, MA), and emulsion and microfluidic sequencing techniques nanodroplets (e.g., GnuBio droplets).

Platforms for next-generation sequencing include, but are not limited to, Roche/454's Genome Sequencer (GS) FLX system, Illumina/Solexa's Genome Analyzer (GA), Life/APG's Support Oligonucleotide Ligation Detection (SOLiD) system, Polonator's G.007 system, Helicos BioSciences' HeliScope Gene Sequencing system, Oxford Nanopore Technologies' PromethION, GrilON, and MinION systems, and Pacific Biosciences' PacBio RS system.

In the present invention, the sequence alignment of step (b) comprises a computerized method or approach used for identity, wherein the read sequence (for example, from next-generation sequencing, e.g., a short-read sequence) in the genome is likely to be derived by evaluating the similarity between the read sequence and a reference sequence in most cases as a computer algorithm. A variety of algorithms can be applied to the sequence alignment problem. Some algorithms are relatively slow, but allow for relatively high specificity. These include, for example, dynamic programming-based algorithms. Dynamic programming is a method of solving complex problems by breaking them down into simpler steps. Other approaches are relatively more efficient, but typically not as thorough. These include, for example, heuristic algorithms and probabilistic methods designed for searching large databases.

Typically, there are two steps in the alignment process: candidate screening and sequence alignment. Candidate screening reduces the search space for sequence alignment from the entire genome for a shorter enumeration of possible alignment positions. Sequence alignment, as the term suggests, involves aligning sequences with sequences provided in the candidate screening step. This can be done using a global alignment (e.g., Needleman-Wunsch alignment) or a local alignment (e.g., Smith-Waterman alignment).

Most attribute sorting algorithms can be characterized as one of three types based on their indexing method: algorithms based on hash tables (e.g., BLAST, ELAND, SOAP), suffix trees (e.g., Bowtie, BWA), and merge alignments (e.g., Slider). Short read sequences are typically used for alignment.

In the present invention, the alignment step in step (b) may be performed using, but not limited to, the BWA algorithm and the Hg19 sequence.

In the present invention, the BWA algorithm may include, but is not limited to, BWA-ALN, BWA-SW, or Bowtie2.

In the present invention, the length of the sequence information (reads) in step (b) is 5 to 5000 bp, and the number of used sequence information can be 50 to 5 million, but is not limited thereto.

The present invention may further comprises the step of selecting reads having an alignment quality score of the aligned nucleic acid fragments above a cut-off value prior to performing step (c), wherein the cut-off value may be any value capable of confirming the quality of the aligned nucleic acid fragments, preferably between 50 and 70, and more preferably 60, but is not limited thereto.

In the present invention, the size of the nucleic acid fragment in step (c) is the number of bases at the 5' end to the 3' end of the nucleic acid fragment.

In the present invention, the size of the nucleic acid fragment in step (c) can be any size that is capable of distinguishing between a healthy person and a cancer patient, and can be preferably 90 to 250 bp, and more preferably selected from the group consisting of, but not limited to, 127 to 129 bp, 137 to 139 bp, 148 to 150 bp, 156 to 158 bp, and 181 to 183 bp.

For example, if there is a nucleic acid fragment that was sequenced by paired-end sequencing as shown below,

Forward strand: 5'-TACAGACTTTGGAAT-3' (SEQ ID No. 1)
Reverse strand: 3'-ATGACTGAAACCTTA-5' (SEQ ID No. 2)

The number of bases at the forward strand 5' end to the 3' end, 15, is the size value of the nucleic acid fragment.

In the present invention, the sequence relative frequency for each position of the nucleic acid fragments in step (c) may be the number of nucleic acid fragments having A, T, G and C bases detected at each position, in nucleic acid fragments of the same size, normalized to the total number of nucleic acid fragments.

In the present invention, the position of the nucleic acid fragment in step (c) can be 1 to 10 bases at the 5' end of the nucleic acid fragment.

In the present invention, the sequence relative frequency for each position of the nucleic acid fragments in step (c) may be the frequency of A, T, G and C bases at positions 1 to 5, and the frequency of A base at positions 6 to 10 at the 5' end of the nucleic acid fragment.

In the present invention, the sequence relative frequency at each position of the nucleic acid fragments and the size of nucleic acid fragments in step (c) may be one or more selected from those listed in Table 3, preferably the sequence relative frequency at each position and the size of nucleic acid fragments of Top 1 to Top 5 listed in Table 7. More preferably, it may be the sequence relative frequency at each position and the size of nucleic acid fragments of up to Top 50 listed in Table 7, and most preferably, it may be the sequence relative frequency at each position and the size of nucleic acid fragments of up to Top 375 listed in Table 7.

[Table 3]

| Feature List | | | | | |
|---|---|---|---|---|---|
| # | Feature | # | Feature | # | Feature |
| 1 | Size127_For1_A | 126 | Size139_For1_A | 251 | Size157_For1_A |
| 2 | Size127_For1_T | 127 | Size139_For1_T | 252 | Size157_For1_T |
| 3 | Size127_For1_G | 128 | Size139_For1_G | 253 | Size157_For1_G |
| 4 | Size127_For1_C | 129 | Size139_For1_C | 254 | Size157_For1_C |
| 5 | Size127_For2_A | 130 | Size139_For2_A | 255 | Size157_For2_A |
| 6 | Size127_For2_T | 131 | Size139_For2_T | 256 | Size157_For2_T |
| 7 | Size127_For2_G | 132 | Size139_For2_G | 257 | Size157_For2_G |

(continued)

| Feature List | | | | | |
|---|---|---|---|---|---|
| # | Feature | # | Feature | # | Feature |
| 8 | Size127_For2_C | 133 | Size139_For2_C | 258 | Size157_For2_C |
| 9 | Size127_For3_A | 134 | Size139_For3_A | 259 | Size157_For3_A |
| 10 | Size127_For3_T | 135 | Size139_For3_T | 260 | Size157_For3_T |
| 11 | Size127_For3_G | 136 | Size139_For3_G | 261 | Size157_For3_G |
| 12 | Size127_For3_C | 137 | Size139_For3_C | 262 | Size157_For3_C |
| 13 | Size127_For4_A | 138 | Size139_For4_A | 263 | Size157_For4_A |
| 14 | Size127_For4_T | 139 | Size139_For4_T | 264 | Size157_For4_T |
| 15 | Size127_For4_G | 140 | Size139_For4_G | 265 | Size157_For4_G |
| 16 | Size127_For4_C | 141 | Size139_For4_C | 266 | Size157_For4_C |
| 17 | Size127_For5_A | 142 | Size139_For5_A | 267 | Size157_For5_A |
| 18 | Size127_For5_T | 143 | Size139_For5_T | 268 | Size157_For5_T |
| 19 | Size127_For5_G | 144 | Size139_For5_G | 269 | Size157_For5_G |
| 20 | Size127_For5_C | 145 | Size139_For5_C | 270 | Size157_For5_C |
| 21 | Size127_For6_A | 146 | Size139_For6_A | 271 | Size157_For6_A |
| 22 | Size127_For7_A | 147 | Size139_For7_A | 272 | Size157_For7_A |
| 23 | Size127_For8_A | 148 | Size139_For8_A | 273 | Size157_For8_A |
| 24 | Size127_For9_A | 149 | Size139_For9_A | 274 | Size157_For9_A |
| 25 | Size127_For10_A | 150 | Size139_For10_A | 275 | Size157_For10_A |
| 26 | Size128_For1_A | 151 | Size148_For1_A | 276 | Size158_For1_A |
| 27 | Size128_For1_T | 152 | Size148_For1_T | 277 | Size158_For1_T |
| 28 | Size128_For1_G | 153 | Size148_For1_G | 278 | Size158_For1_G |
| 29 | Size128_For1_C | 154 | Size148_For1_C | 279 | Size158_For1_C |
| 30 | Size128_For2_A | 155 | Size148_For2_A | 280 | Size158_For2_A |
| 31 | Size128_For2_T | 156 | Size148_For2_T | 281 | Size158_For2_T |
| 32 | Size128_For2_G | 157 | Size148_For2_G | 282 | Size158_For2_G |
| 33 | Size128_For2_C | 158 | Size148_For2_C | 283 | Size158_For2_C |
| 34 | Size128_For3_A | 159 | Size148_For3_A | 284 | Size158_For3_A |
| 35 | Size128_For3_T | 160 | Size148_For3_T | 285 | Size158_For3_T |
| 36 | Size128_For3_G | 161 | Size148_For3_G | 286 | Size158_For3_G |
| 37 | Size128_For3_C | 162 | Size148_For3_C | 287 | Size158_For3_C |
| 38 | Size128_For4_A | 163 | Size148_For4_A | 288 | Size158_For4_A |
| 39 | Size128_For4_T | 164 | Size148_For4_T | 289 | Size158_For4_T |
| 40 | Size128_For4_G | 165 | Size148_For4_G | 290 | Size158_For4_G |
| 41 | Size128_For4_C | 166 | Size148_For4_C | 291 | Size158_For4_C |
| 42 | Size128_For5_A | 167 | Size148_For5_A | 292 | Size158_For5_A |
| 43 | Size128_For5_T | 168 | Size148_For5_T | 293 | Size158_For5_T |
| 44 | Size128_For5_G | 169 | Size148_For5_G | 294 | Size158_For5_G |

(continued)

| Feature List | | | | | |
|---|---|---|---|---|---|
| # | Feature | # | Feature | # | Feature |
| 45 | Size128_For5_C | 170 | Size148_For5_C | 295 | Size158_For5_C |
| 46 | Size128_For6_A | 171 | Size148_For6_A | 296 | Size158_For6_A |
| 47 | Size128_For7_A | 172 | Size148_For7_A | 297 | Size158_For7_A |
| 48 | Size128_For8_A | 173 | Size148_For8_A | 298 | Size158_For8_A |
| 49 | Size128_For9_A | 174 | Size148_For9_A | 299 | Size158_For9_A |
| 50 | Size128_For10_A | 175 | Size148_For10_A | 300 | Size158_For10_A |
| 51 | Size129_For1_A | 176 | Size149_For1_A | 301 | Size181_For1_A |
| 52 | Size129_For1_T | 177 | Size149_For1_T | 302 | Size181_For1_T |
| 53 | Size129_For1_G | 178 | Size149_For1_G | 303 | Size181_For1_G |
| 54 | Size129_For1_C | 179 | Size149_For1_C | 304 | Size181_For1_C |
| 55 | Size129_For2_A | 180 | Size149_For2_A | 305 | Size181_For2_A |
| 56 | Size129_For2_T | 181 | Size149_For2_T | 306 | Size181_For2_T |
| 57 | Size129_For2_G | 182 | Size149_For2_G | 307 | Size181_For2_G |
| 58 | Size129_For2_C | 183 | Size149_For2_C | 308 | Size181_For2_C |
| 59 | Size129_For3_A | 184 | Size149_For3_A | 309 | Size181_For3_A |
| 60 | Size129_For3_T | 185 | Size149_For3_T | 310 | Size181_For3_T |
| 61 | Size129_For3_G | 186 | Size149_For3_G | 311 | Size181_For3_G |
| 62 | Size129_For3_C | 187 | Size149_For3_C | 312 | Size181_For3_C |
| 63 | Size129_For4_A | 188 | Size149_For4_A | 313 | Size181_For4_A |
| 64 | Size129_For4_T | 189 | Size149_For4_T | 314 | Size181_For4_T |
| 65 | Size129_For4_G | 190 | Size149_For4_G | 315 | Size181_For4_G |
| 66 | Size129_For4_C | 191 | Size149_For4_C | 316 | Size181_For4_C |
| 67 | Size129_For5_A | 192 | Size149_For5_A | 317 | Size181_For5_A |
| 68 | Size129_For5_T | 193 | Size149_For5_T | 318 | Size181_For5_T |
| 69 | Size129_For5_G | 194 | Size149_For5_G | 319 | Size181_For5_G |
| 70 | Size129_For5_C | 195 | Size149_For5_C | 320 | Size181_For5_C |
| 71 | Size129_For6_A | 196 | Size149_For6_A | 321 | Size181_For6_A |
| 72 | Size129_For7_A | 197 | Size149_For7_A | 322 | Size181_For7_A |
| 73 | Size129_For8_A | 198 | Size149_For8_A | 323 | Size181_For8_A |
| 74 | Size129_For9_A | 199 | Size149_For9_A | 324 | Size181_For9_A |
| 75 | Size129_For10_A | 200 | Size149_For10_A | 325 | Size181_For10_A |
| 76 | Size137_For1_A | 201 | Size150_For1_A | 326 | Size182_For1_A |
| 77 | Size137_For1_T | 202 | Size150_For1_T | 327 | Size182_For1_T |
| 78 | Size137_For1_G | 203 | Size150_For1_G | 328 | Size182_For1_G |
| 79 | Size137_For1_C | 204 | Size150_For1_C | 329 | Size182_For1_C |
| 80 | Size137_For2_A | 205 | Size150_For2_A | 330 | Size182_For2_A |
| 81 | Size137_For2_T | 206 | Size150_For2_T | 331 | Size182_For2_T |

(continued)

| Feature List | | | | | |
|---|---|---|---|---|---|
| # | Feature | # | Feature | # | Feature |
| 82 | Size137_For2_G | 207 | Size150_For2_G | 332 | Size182_For2_G |
| 83 | Size137_For2_C | 208 | Size150_For2_C | 333 | Size182_For2_C |
| 84 | Size137_For3_A | 209 | Size150_For3_A | 334 | Size182_For3_A |
| 85 | Size137_For3_T | 210 | Size150_For3_T | 335 | Size182_For3_T |
| 86 | Size137_For3_G | 211 | Size150_For3_G | 336 | Size182_For3_G |
| 87 | Size137_For3_C | 212 | Size150_For3_C | 337 | Size182_For3_C |
| 88 | Size137_For4_A | 213 | Size150_For4_A | 338 | Size182_For4_A |
| 89 | Size137_For4_T | 214 | Size150_For4_T | 339 | Size182_For4_T |
| 90 | Size137_For4_G | 215 | Size150_For4_G | 340 | Size182_For4_G |
| 91 | Size137_For4_C | 216 | Size150_For4_C | 341 | Size182_For4_C |
| 92 | Size137_For5_A | 217 | Size150_For5_A | 342 | Size182_For5_A |
| 93 | Size137_For5_T | 218 | Size150_For5_T | 343 | Size182_For5_T |
| 94 | Size137_For5_G | 219 | Size150_For5_G | 344 | Size182_For5_G |
| 95 | Size137_For5_C | 220 | Size150_For5_C | 345 | Size182_For5_C |
| 96 | Size137_For6_A | 221 | Size150_For6_A | 346 | Size182_For6_A |
| 97 | Size137_For7_A | 222 | Size150_For7_A | 347 | Size182_For7_A |
| 98 | Size137_For8_A | 223 | Size150_For8_A | 348 | Size182_For8_A |
| 99 | Size137_For9_A | 224 | Size150_For9_A | 349 | Size182_For9_A |
| 100 | Size137_For10_A | 225 | Size150_For10_A | 350 | Size182_For10_A |
| 101 | Size138_For1_A | 226 | Size156_For1_A | 351 | Size183_For1_A |
| 102 | Size138_For1_T | 227 | Size156_For1_T | 352 | Size183_For1_T |
| 103 | Size138_For1_G | 228 | Size156_For1_G | 353 | Size183_For1_G |
| 104 | Size138_For1_C | 229 | Size156_For1_C | 354 | Size183_For1_C |
| 105 | Size138_For2_A | 230 | Size156_For2_A | 355 | Size183_For2_A |
| 106 | Size138_For2_T | 231 | Size156_For2_T | 356 | Size183_For2_T |
| 107 | Size138_For2_G | 232 | Size156_For2_G | 357 | Size183_For2_G |
| 108 | Size138_For2_C | 233 | Size156_For2_C | 358 | Size183_For2_C |
| 109 | Size138_For3_A | 234 | Size156_For3_A | 359 | Size183_For3_A |
| 110 | Size138_For3_T | 235 | Size156_For3_T | 360 | Size183_For3_T |
| 111 | Size138_For3_G | 236 | Size156_For3_G | 361 | Size183_For3_G |
| 112 | Size138_For3_C | 237 | Size156_For3_C | 362 | Size183_For3_C |
| 113 | Size138_For4_A | 238 | Size156_For4_A | 363 | Size183_For4_A |
| 114 | Size138_For4_T | 239 | Size156_For4_T | 364 | Size183_For4_T |
| 115 | Size138_For4_G | 240 | Size156_For4_G | 365 | Size183_For4_G |
| 116 | Size138_For4_C | 241 | Size156_For4_C | 366 | Size183_For4_C |
| 117 | Size138_For5_A | 242 | Size156_For5_A | 367 | Size183_For5_A |
| 118 | Size138_For5_T | 243 | Size156_For5_T | 368 | Size183_For5_T |

(continued)

| Feature List | | | | | |
| --- | --- | --- | --- | --- | --- |
| # | Feature | # | Feature | # | Feature |
| 119 | Size138_For5_G | 244 | Size156_For5_G | 369 | Size183_For5_G |
| 120 | Size138_For5_C | 245 | Size156_For5_C | 370 | Size183_For5_C |
| 121 | Size138_For6_A | 246 | Size156_For6_A | 371 | Size183_For6_A |
| 122 | Size138_For7_A | 247 | Size156_For7_A | 372 | Size183_For7_A |
| 123 | Size138_For8_A | 248 | Size156_For8_A | 373 | Size183_For8_A |
| 124 | Size138_For9_A | 249 | Size156_For9_A | 374 | Size183_For9_A |
| 125 | Size138_For10_A | 250 | Size156_For10_A | 375 | Size183_For10_A |

In the present invention, the position of the nucleic acid fragment is defined based on the 5' end of the nucleic acid fragment.

For example, the position of the nucleic acid fragment from the 5' end of the forward strand of SEQ ID No. 1 can have a value of For1, For2, ... or For 15, and the same for the reverse strand. The value of For1 of SEQ ID No. 1 is T, and the value of Rev1 of the reverse strand is A.

In the present invention, the frequency of a base sequence at each position of a nucleic acid fragment can be calculated by the following steps:

a) separating the whole nucleic acid fragment into a group of nucleic acid fragments with the same size;
b) counting the number of A, T, G, and C bases by nucleic acid fragment position within each group; and
c) normalizing the number of bases by nucleic acid fragment position using Formula 2.

$$\text{Formula 2: Size120\_For1\_A} = \frac{\# \ of \ fragments \ with \ size=120, position=For1, base=A}{\# \ of \ all \ reads \ sequenced}$$

In the present invention, it is apparent to those skilled in the art that the size, position and base in Formula 2 vary depending on the size, position and base to be normalized.

In the present invention, the artificial intelligence model in step (d) can be any model that can be trained to discriminate between healthy people and cancer patients, preferably a machine learning model.

In the present invention, the artificial intelligence model may be selected from the group consisting of, but not limited to, AdaBoost, Random forest, Catboost, Light Gradient Boosting Model, and XGBoost.

In the present invention, when the artificial intelligence model is XGBoost and learns binary classification, the loss function is represented by Formula 1 below.

Formula 1: Binary classification

$$\text{loss}(\text{model}(x), y) = -\frac{1}{n} \left[ \sum_{i=1}^{n} (y_i \log(model(x_i)) + (1 - y_i) \log(1 - model(x_i))) \right]$$

model($x_i$) = *Output of the XGboost model f or the $i_{th}$ input*
y = Actual label value
n = Number of input data

In the present invention, the binary classification means training an artificial intelligence model to determine the presence or absence of cancer.

In the present invention, when the artificial intelligence model is XGBoost, the training may be performed including the following steps:

i) categorizing the sequence relative frequency and the size information for each position of nucleic acid fragments into training, validation, and test data,

wherein the training data is used to train the XGBoost model, validation data is used to validate hyper-parameter tuning, and test data is used for performance evaluation after producing the optimal model;

ii) building an optimal XGBoost model through hyper-parameter tuning and learning process; and

iii) comparing the performance of multiple models obtained through hyper-parameter tuning by using validation data to determine the model with the best validation data performance as the optimal model.

In the present invention, the hyper-parameter tuning process is a process of optimizing the various parameters (i.e., maximum depth of learner tree, number of learner trees, learning rate, etc.) comprised in the XGBoost model, and the hyper-parameter tuning process may use Bayesian optimization and grid search techniques.

In the present invention, the learning process may be characterized by optimizing the internal parameters (weights) of the XGBoost model by using a set of hyper-parameters, and determining that the model is overfitted when the validation loss starts to increase compared to the training loss, and stopping the model training before that.

In the present invention, the resultant value of the analysis from the sequence relative frequency and size information at each position of the nucleic acid fragment with the artificial intelligence model input in step d) may be any specific score or real number, preferably an XPI (XGBoost Probability Index) value, but is not limited thereto.

In the present invention, XGBoost Probability Index means a value expressed as a probability value by adjusting the output of an artificial intelligence model to a scale of 0 to 1.

For binary classification, the sigmoid function is used to learn that the XPI value is 1 for cancer. For example, if a neuroblastoma sample and a normal sample are input, it is trained so that the XPI value of the neuroblastoma sample is close to 1 and the normal sample is close to 0.

In the present invention, when the artificial intelligence model is trained, the output result is trained to be close to 1 if there is cancer, and the output result is trained to be close to 0 if there is no cancer, and the performance measurement is performed by judging that there is cancer if the output result is above 0.5, and there is no cancer if the output result is below 0.5 (training, validation, test accuracy).

Here, it is apparent to one of ordinary skill in the art that the cut-off value of 0.5 is a value that can be changed at any time. For example, if it is desired to reduce false positives, the cut-off value higher than 0.5 can be set to set strict criteria for determining if there is cancer, and if it is desired to reduce false negatives, the lower cut-off value can be set to set loose criteria for determining if there is cancer.

Most preferably, a trained artificial intelligence model can be used to apply unseen data (data that knows the answer which was not trained) to determine the probability of an XPI value to establish a cut-off value.

In another aspect, the present invention provides a cancer diagnostic device comprising: a decoding part that extracts nucleic acid from a biospecimen and decodes sequence information; an alignment part that aligns the decoded sequence to a reference genome database; a nucleic acid fragment analysis part that derives the sequence relative frequency at each position of the nucleic acid fragment and the size of the nucleic acid fragment based on the aligned sequence; and a cancer diagnostic part that inputs the derived sequence relative frequency at each position of the nucleic acid fragment and size information of the nucleic acid fragment into a trained artificial intelligence model, analyzes same, and determines the presence of cancer by comparing the analyzed output to a cut-off value.

In the present invention, the decoding part may include a nucleic acid injection part for injecting nucleic acid extracted from an independent device; and a sequence information analysis part for analyzing sequence information of the injected nucleic acid, which may preferably be an NGS analysis device, but is not limited thereto.

In the present invention, the decoding part may receive and decode sequence information data generated by an independent device.

In another aspect, the present invention provides a computer-readable storage medium comprising instructions configured to be executed by a processor to provide information for diagnosing cancer, the instructions comprising (a) obtaining a sequence information from extracted nucleic acid from a biological sample; (b) aligning the obtained sequence information (reads) to a reference genome database; (c) deriving the sequence relative frequency and the size at each position of nucleic acid fragments using the aligned sequence information (reads); and (d) inputting the derived sequence relative frequency and size information to an artificial intelligence model trained to diagnose cancer and comparing the analyzed output with a cut-off value to determine the presence or absence of cancer, wherein the artificial intelligence model is trained to distinguish normal samples and cancer samples based on the sequence relative frequency at each position of the nucleic acid fragment and the size information of the nucleic acid fragment.

In another aspect, the present invention relates to a method of diagnosing cancer using a cell-free nucleic acid, comprising the following steps:

(a) obtaining a sequence information from extracted nucleic acid from a biological sample;
(b) aligning the obtained sequence information (reads) to a reference genome database;
(c) using the aligned sequence information (reads) to derive the sequence relative frequency at each position of the nucleic acid fragment and the size of nucleic acid fragment; and

(d) inputting the derived sequence relative frequency and size information into an artificial intelligence model trained to diagnose cancer, and comparing the analyzed output to a cut-off value to determine the presence or absence of cancer,

wherein the artificial intelligence model is trained to distinguish normal samples and cancer samples based on the sequence relative frequency at each position of the nucleic acid fragment and the size information of the nucleic acid fragment.

In another aspect, the method according to the present invention may be implemented by using a computer. In one embodiment, the computer includes one or more processors coupled to a chip set. Also connected to the chip set are memory, storage devices, a keyboard, a graphics adapter, a pointing device, and a network adapter. In one embodiment, the performance of the chip set is enabled by a memory controller hub and an I/O controller hub. In other embodiment, the memory may be directly connected to a processor instead of a chip set. The storage device is any device capable of holding data, including a hard drive, compact disk read-only memory (CD-ROM), DVD, or other memory device. The memory is involved in the data and instructions used by the processor. The pointing device may be a mouse, track ball, or other type of pointing device, and is used in combination with a keyboard to transmit input data to the computer system. A graphics adapter represents images and other information on a display. The network adapter is connected to the computer system by a local area or long distance communication network. The computers used herein are not limited to the above configurations, however, and may include some or additional configurations, and may also be part of a storage area network (SAN), and the computers may be configured to be suitable for executing modules in a program for performing the methods according to the present invention.

As used herein, a module may refer to a functional and structural combination of hardware for performing a technical idea according to the present invention and software for operating the hardware. For example, it is apparent to a person skilled in the art that a module may refer to a logical unit of predetermined code and a hardware resource on which the predetermined code is performed, and does not necessarily refer to physically connected code or a type of hardware.

Methods according to the present invention may be implemented in hardware, firmware, or software, or a combination thereof. When implemented as software, the storage medium includes any medium for storing or communicating information in a form readable by a device such as a computer. For example, computer-readable media include read only memory (ROM); random access memory (RAM); magnetic disk storage media; optical storage media; flash memory devices; and other electrical, optical, or acoustic signal transmission media.

## Example

The present invention is described in more detail by providing the following examples. These examples are intended solely to illustrate the present invention, and it would be apparent to one of ordinary skill in the art that the scope of the present invention is not to be construed as limited by these examples.

### Example 1. Extracting DNA from blood for next-generation sequencing

10 ml of Blood from 202 healthy individuals and 61 neuroblastoma patients was stored in EDTA tubes, and within 2 hours after collection, only the plasma portion was subjected to primary centrifugation in an amount of 1200g, at 4°C, for 15 minutes, and then the primary centrifuged plasma was subjected to secondary centrifugation in an amount of 16000g, at 4°C, for 10 minutes to separate the plasma supernatant excluding sediment. For the separated plasma, cell-free DNA was extracted using Chemagic ccfNA 2K Kit (chemagen), library preparation was performed using MGIEasy cell-free DNA library prep set kit, and then sequencing using DNBseq G400 instrument (MGI) in 100 base paired end mode was performed. As a result, it was found that about 1.7 million reads were produced per sample.

### Example 2. Screening for optimal sequence relative frequency for each position and size of nucleic acid fragment

2-1. Defining and measuring the nucleic acid fragment position and the relative frequency of base sequence

The position of the nucleic acid fragment was defined based on the 5' end of the nucleic acid fragment.

Since the reads obtained in Example 1 are paired-end sequencing reads and are 100 bp long, the forward strand was positioned at the 5' end to For1, For2, ..., For 100, and the reverse strand was positioned at the 5' end to Rev1, Rev2, ..., Rev 100. The nucleic acid fragments were assembled using the bamtobed -bedpe option of the bedtools program.

To briefly describe the process of obtaining the relative frequency of base sequence by position of nucleic acid fragments, first, from the sequencing data of about 170M reads produced in Example 1, 17M reads were randomly selected and downsampled, then QC filtering was performed, the number of fragments satisfying the combination of size, position, and base (ex, Size120_For1_A) was counted, and normalization was performed by dividing by the total number of sequencing reads remaining after the above 3 QC filtering.

More specifically, the process was performed by the following steps:

1. The total nucleic acid fragments were divided into groups of nucleic acid fragments with the same size, for example, a group of nucleic acid fragments with size 101, a group of 150, a group of ... 200, etc.

2. The number of A, T, G and C bases for each position of nucleic acid fragment in each group was counted. For example, the number of bases for each position of nucleic acid fragment in the group with a nucleic acid fragment size of 120 was counted and summarized as shown in Table 1 below.

[Table 1]

| Size= 120 | For1 | For2 | For3 | ... | For100 | Rev100 | ... | Rev3 | Rev2 | Rev1 |
|---|---|---|---|---|---|---|---|---|---|---|
| A | 5,683 | 6,999 | 6,694 | ... | 6,998 | 6,429 | ... | 6,807 | 6,942 | 5,619 |
| T | 4,680 | 4,422 | 8,283 | ... | 7,825 | 7,986 | ... | 8,329 | 4,438 | 4,716 |
| G | 4,194 | 5,555 | 2,730 | ... | 3,970 | 3,952 | ... | 2,772 | 5,609 | 4,111 |
| C | 8,566 | 6,153 | 5,413 | ... | 4,336 | 4,768 | ... | 5,219 | 6,142 | 8,676 |
| N | 12 | 6 | 15 | ... | 6 | - | ... | 8 | 4 | 13 |
| Sum | 23,135 | 23,135 | 23,135 | ... | 23,135 | 23,135 | ... | 23,135 | 23,135 | 23,135 |

Interpreting the table above, it means that there were a total of 23,135 nucleic acid fragments of size 120, of which 5,683, 4,680, 4,194, and 8,566 had A, T, G, and C bases in the For1 position, respectively.

3. After counting the number of bases for each position of nucleic acid fragment by the above process, the total number of sequenced reads (the number of all reads of the produced analytes, regardless of nucleic acid fragment size. In Example 1, 15,063,130) was divided by the total number of sequenced reads and normalized by Formula 2 to produce a FESS (Fragment End Sequence frequency and Size) table calculating the relative frequency as shown in Table 2 (FESS_Table_120) below.

$$\text{Formula 2: Size120\_For1\_A} = \frac{\#\ of\ fragments\ with\ size=120, position=For1, base=A}{\#\ of\ all\ reads\ sequenced}$$

[Table 2]

| Size=120 | For1 | For2 | For3 | ... | For100 | Rev100 | ... | Rev3 | Rev2 | Rev1 |
|---|---|---|---|---|---|---|---|---|---|---|
| A | 0.000377 | 0.000465 | 0.000444 | ... | 0.000465 | 0.000427 | ... | 0.000452 | 0.000461 | 0.000373 |
| T | 0.000311 | 0.000294 | 0.000550 | ... | 0.000519 | 0.000530 | ... | 0.000553 | 0.000295 | 0.000313 |
| G | 0.000278 | 0.000369 | 0000181 | ... | 0.000264 | 0.000262 | ... | 0.000184 | 0.000372 | 0.000273 |
| C | 0.000569 | 0.000408 | 0.000359 | ... | 0.000288 | 0.000317 | ... | 0.000346 | 0.000408 | 0000576 |

4. The relative frequency of values of N (when a base sequence was not measurable due to sequencing error, low quality, etc.) was not calculated.

2-2. Screening the optimal nucleic acid fragment size

The position and base sequence of the nucleic acid fragment to be analyzed were fixed as (For1_A) and the following analysis was performed.

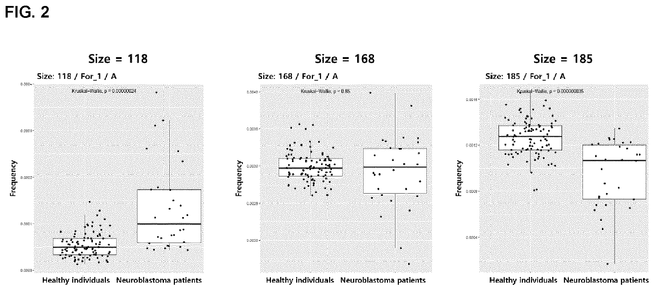

1. The Kruskal-Wallis test was used to statistically determine whether there was a difference in the relative frequency distribution of (For1_A) between healthy individuals and neuroblastoma patients as the nucleic acid fragment size was varied by 1. That is, as shown in FIG. 2, in the nucleic acid fragment group of size 118, the relative frequency of (For1_A) was statistically significantly higher in neuroblastoma patients than in healthy individuals. Similarly, in the nucleic acid fragment group of size **168,** the relative frequency of (For1_A) is not significantly different in the two groups, and in the nucleic acid fragment group of size **185,** the relative frequency of (For1_A) is statistically significantly lower in healthy individuals than in neuroblastoma patients.

2. In this way, the difference in relative frequency of (For1_A) between healthy individuals and neuroblastoma patients was determined statistically (p-value) by varying the nucleic acid fragment size of 101 to 200.

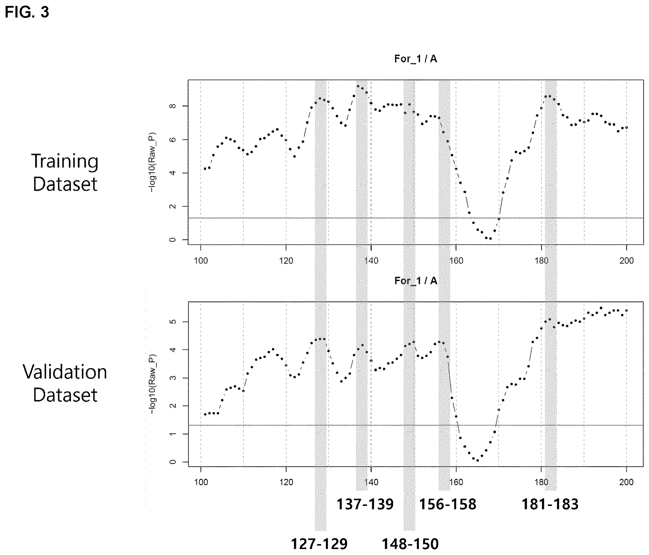

As a result, the x-axis in FIG. 3 represents the nucleic acid fragment size and the y-axis represents the $-\log_{10}(p)$ value, where the larger the y-axis value, the greater the difference between healthy individuals and neuroblastoma patients. As shown in FIG. 3, for a nucleic acid fragment size of about 10, the difference in frequency of (For1_A) between healthy individuals and neuroblastoma patients is significantly wider (the $-\log_{10}(p)$ value peaks and declines).

It is also seen that these patterns are repeated not only in the Training Dataset, but also in the independent Validation Dataset, confirming that the nucleic acid fragment sizes plotted in green are not accidental patterns of overfitting in the Training Dataset.

The nucleic acid fragment sizes with a peak in the value of $-\log_{10}(p)$ in both datasets (127 to 129, 137 to 139, 148 to 150, 156 to 158, 181 to 183), a total of 15 nucleic acid fragment sizes, were selected.

Furthermore, a similar pattern was observed in other bases at other positions (FIG. 9).

2-3. Screening the optimal nucleic acid fragment position

Since the data obtained in Example 1 is 100 PE data, there are a total of 200 nucleic acid fragment positions available for analysis, i.e., For1 to 100 and Rev1 to 100.

FIG. 4 shows a heatmap visualization of *FESS_Table_120* of Table 2, where differences in the relative frequency of A, T, G and C sequences by position are observed only some portions at the dotted ends (For1 to 10, Rev1 to 10), and the relative frequency of nearly similar A, T, G and C base sequences repeats towards the end of the read (to 100).

For example, the relative frequency of A, T, G and C sequences in For1 is significantly different from the relative frequency of A, T, G and C in For2, but the relative frequency of A, T, G and C sequences in For11, the relative frequency of A, T, G and C sequences in For99, and the relative frequency of A, T, G and C sequences in For100 are very similar without significant differences.

Therefore, to improve the performance of the learning model, only For1 to 10 and Rev1 to 10 positions, excluding positions at the end of the read, were selected as features to train the model.

Furthermore, the dotted region in FIG. 4 is enlarged in FIG. 5 (Rev1 to 10 are sorted in reverse order of Rev10 to 1), it is seen that in the leftmost panel that the relative frequencies of the same sequences in the same position in forward and reverse are quite similar.

For example, (For1_A and Rev1_A), (For1_T and Rev1_T), (For1_G and Rev1_G), and (For1_C and Rev1_C) have similar relative frequency values, and in the same way, (For2_A and Rev2_A), (For2_T and Rev2_T), (For2_G and Rev2_G), and (For2_C and Rev2_C) have similar relative frequency values.

This similarity was measured by Pearson's correlation coefficient in the healthy group, as shown in the two right panels of FIG. 5. It was found that the similarity between the relative frequency values of For1_A and the relative frequency values of Rev1_A, the similarity between the relative frequency values of For1_T and the relative frequency values of Rev1_T, the similarity between the relative frequency values of For1_G and the relative frequency values of Rev1_G, and the similarity between the relative frequency values of For1_C and the relative frequency values of Rev1_C measured in the healthy group are all equal to 1.

Through this analysis, it was found that the relative frequency of the 5' end sequence on the forward strand of the nucleic acid fragment was similar to the relative frequency of the 5' end sequence on the reverse strand, so only the For1 to 10 positions, excluding the Rev1 to 10 positions, were selected as features to train the model.

2-4. Screening the optimal nucleic acid fragment for each position

At each of the 10 positions selected in Examples 2 and 3, the relative frequency of the four types of sequences, A, T, G, and C can be calculated. For example, at position For1, the relative frequencies of For1_A, For1_T, For1_G, and For1_C can be calculated. To reduce the number of variables to train the model, additional screening was performed by checking for similarities between sequences in the same position. Sequence selection for each position was performed in a healthy group using the following method.

1. For1-10 positions, the relative frequency of A, T, G, and C base sequences at each position was calculated,

2. The similarity between (For1_A and For1_T), (For1_A and For1_G), (For1_A and For1_C), (For1_T and For1_G), (For1_T and For1_C), and (For1_G and For1_C) was measured by Pearson's correlation coefficient.

As a result, as shown in FIG. 6, it was found that there is a low similarity between the relative frequencies of the four types of base sequences A, T, G and C at positions For1 to 5, and there is a significantly higher similarity between the relative frequencies of the four types of sequences A, T, G and C at positions For6 to 10.

Therefore, all four types of sequences (A, T, G and C) were selected in positions For1 to For5, and only the A sequence was selected as a representative value among A, T, C and G in positions For6 to For10.

In conclusion, the optimal nucleic acid fragment size and relative frequency of sequences for each position are given below:

1) Nucleic acid fragment sizes: 127, 128, 129, 137, 138, 139, 148, 149, 150, 156, 157, 158, 181, 182, 183. 15 in total.

2) Nucleic acid fragment positions: For1 to 10. 10 in total.

3) Sequence combinations for each nucleic acid fragment position

For1 to 5: A, T, G, C For6 to 10: A

15 sizes * 25 positions base sequences = 375 features

The 375 feature combinations are listed in Table 3.

## Example 3. Building and training a machine learning model

Using the relative frequency values of the 375 features selected in Example 2 as input, a machine learning model was trained to distinguish between healthy individuals and neuroblastoma patients. XGBoost was used as the machine learning algorithm.

The total sample was divided into Training, Validation, and Test datasets, with the Training dataset used for model training, the Validation dataset used for hyper-parameter tuning, and the Test dataset used for final model performance evaluation. The number of samples in each set is shown below.

[Table 4]

| Dataset | Healthy individuals | Neuroblastoma patients | Total |
|---------|---------------------|------------------------|-------|
| Train | 99 | 30 | 129 |
| Valid | 42 | 13 | 55 |
| Test | 61 | 18 | 79 |
| Total | 202 | 61 | 263 |

Hyper-parameter tuning is the process of optimizing the values of various parameters (maximum depth of learner tree, number of learner trees, learning rate, etc.) that make up the XGBoost model.

Bayesian optimization and grid search techniques were used for hyper-parameter tuning, and model training was stopped when the training loss and validation loss began to increase, indicating that the model was overfitting.

The performance of various models obtained through hyper-parameter tuning was compared using the validation data set, and the model with the best performance on the validation data set was judged as the optimal model, and the final performance evaluation was performed on the test data set.

When the XGBoost model created through the above process is inputted with a vector of relative frequency values of 375 features calculated from a random sample, the probability of the sample being a healthy person or a neuroblastoma patient is calculated, and this probability value is defined as the XGBoost Probability Index (XPI).

If the XPI value calculated for a random sample was greater than or equal to 0.5, it was considered a neuroblastoma patient, and if it was less than or equal to 0.5, it was considered healthy.

**Example 4. Verifying the performance of the built model**

4-1 Verifying the performance

The performance of the XPI value output by the machine learning model built in Example 3. All samples were divided into Train, Validation, and Test groups, and the model was built using the Train samples, and then the performance of the model built using the Train samples was checked using the samples from the Validation and Test groups.

[Table 5]

|  | Accuracy | AUC |
|---|---|---|
| **Train** | 1.000 | 1.000 |
| **Validation** | 0.945 | 0.952 |
| **Test** | 0.937 | 0.987 |

As a result, as shown in Table 5 and FIG. 7, Accuracy was found to be 1.000, 0.945, and 0.937 for Train, Valid, and Test groups, respectively, and AUC values were found to be 1.000, 0.952, and 0.987 for Train, Valid, and Test groups, respectively, based on ROC analysis.

4-2. Checking XPI distribution

The XPI values, which are the output of the machine learning model built in Example 3, were checked to see how well they match the actual patients. In FIG. 8, the x-axis represents the group (True label) information of the actual sample, and the y-axis represents the XPI values calculated by the machine learning model for a healthy person (Normal), a neuroblastoma patient (NBT), in order from left to right.
As a result, the XPI distribution, as shown in FIG. 8, shows that the healthy samples in all of the Train, Validation, and Test datasets are most likely to be healthy people, while the neuroblastoma patient samples are most likely to be liver cancer patients.

Example 5. Checking model performance by feature

5-1 Deriving importance by feature

The features selected in Example 2 were used to build the learning model in Example 3, and when the XGB model was trained by using each feature, the importance values of each feature were as shown in Table 6 below.

[Table 6]

| Importance by feature | | | | | |
|---|---|---|---|---|---|
| **Rank** | **Feature** | **Importance** | **Rank** | **Feature** | **Importance** |
| 1 | Size_1 49_For_5_G | 0.093 | 189 | Size_138_For_5_A | 0.000 |
| 2 | Size_128_For_1_A | 0.071 | 190 | Size_138_For_5_T | 0.000 |
| 3 | Size_1_38_For_1_G | 0.054 | 191 | Size_138_For_5_G | 0.000 |
| 4 | Size_182_For_4_T | 0.052 | 192 | Size_138_For_5_C | 0.000 |
| 5 | Size_157_For_1_A | 0.032 | 193 | Size_138_For_6_A | 0.000 |
| 6 | Size_127_For_1_A | 0.031 | 194 | Size_138_For_7_A | 0.000 |
| 7 | Size_158_For_1_A | 0.029 | 195 | Size_138_For_8_A | 0.000 |
| 8 | Size_137_For_7_A | 0.029 | 196 | Size_138_For_9_A | 0.000 |
| 9 | Size_156_For_5_G | 0.024 | 197 | Size_138_For_10_A | 0.000 |
| 10 | Size_182_For_5_T | 0.024 | 198 | Size_139_For_1_A | 0.000 |
| 11 | Size_127_For_2_C | 0.024 | 199 | Size_139_For_1_T | 0.000 |

(continued)

| Importance by feature | | | | | |
|---|---|---|---|---|---|
| Rank | Feature | Importance | Rank | Feature | Importance |
| 12 | Size_139_For_2_A | 0.023 | 200 | Size_139_For_1_G | 0.000 |
| 13 | Size_183_For_3_G | 0.023 | 201 | Size_139_For_1_C | 0.000 |
| 14 | Size_181_For_5_T | 0.020 | 202 | Size_139_For_2_T | 0.000 |
| 15 | Size_148_For_1_G | 0.020 | 203 | Size_139_For_2_G | 0.000 |
| 16 | Size_156_For_1_G | 0.019 | 204 | Size_139_For_2_C | 0.000 |
| 17 | Size_150_For_9_A | 0.018 | 205 | Size_139_For_3_A | 0.000 |
| 18 | Size_127_For_5_G | 0.018 | 206 | Size_139_For_3_T | 0.000 |
| 19 | Size_183_For_1_T | 0.017 | 207 | Size_139_For_3_G | 0.000 |
| 20 | Size_181_For_1_G | 0.016 | 208 | Size_139_For_4_A | 0.000 |
| 21 | Size_137_For_2_T | 0.016 | 209 | Size_139_For_4_T | 0.000 |
| 22 | Size_182_For_8_A | 0.015 | 210 | Size_139_For_4_G | 0.000 |
| 23 | Size_156_For_1_T | 0.013 | 211 | Size_139_For_4_C | 0.000 |
| 24 | Size_158_For_3_T | 0.012 | 212 | Size_139_For_5_A | 0.000 |
| 25 | Size_157_For_2_T | 0.012 | 213 | Size_139_For_5_T | 0.000 |
| 26 | Size_137_For_1_A | 0.011 | 214 | Size_139_For_5_G | 0.000 |
| 27 | Size_150_For_2_C | 0.010 | 215 | Size_139_For_5_C | 0.000 |
| 28 | Size_181_For_3_G | 0.010 | 216 | Size_139_For_6_A | 0.000 |
| 29 | Size_127_For_3_G | 0.010 | 217 | Size_139_For_7_A | 0.000 |
| 30 | Size_156_For_1_C | 0.010 | 218 | Size_139_For_8_A | 0.000 |
| 31 | Size_156_For_4_G | 0.009 | 219 | Size_139_For_9_A | 0.000 |
| 32 | Size_127_For_1_C | 0.009 | 220 | Size_139_For_10_A | 0.000 |
| 33 | Size_156_For_2_T | 0.009 | 221 | Size_148_For_1_A | 0.000 |
| 34 | Size_138_For_3_G | 0.009 | 222 | Size_148_For_1_T | 0.000 |
| 35 | Size_182 _For_1_A | 0.009 | 223 | Size_148_For_1_C | 0.000 |
| 36 | Size_157_For_3_T | 0.009 | 224 | Size_148_For_2_A | 0.000 |
| 37 | Size_156_For_3_T | 0.008 | 225 | Size_148_For_2_T | 0.000 |
| 38 | Size_158_For_1_C | 0.008 | 226 | Size_148_For_2_G | 0.000 |
| 39 | Size_158_For_2_G | 0.007 | 227 | Size_148_For_3_A | 0.000 |
| 40 | Size_158_For_2_C | 0.007 | 228 | Size_148_For_3_T | 0.000 |
| 41 | Size_182_For_5_C | 0.007 | 229 | Size_148_For_3_G | 0.000 |
| 42 | Size_158_For_1_T | 0.006 | 230 | Size_148_For_3_C | 0.000 |
| 43 | Size_149_For_1_G | 0.006 | 231 | Size_148_For_4_A | 0.000 |
| 44 | Size_156_For_4_C | 0.006 | 232 | Size_148_For_4_T | 0.000 |
| 45 | Size_158_For_1_G | 0.006 | 233 | Size_148_For_4_C | 0.000 |
| 46 | Size_157_For_3_A | 0.006 | 234 | Size_148_For_5_A | 0.000 |
| 47 | Size_157_For_2_A | 0.005 | 235 | Size_148_For_5_T | 0.000 |
| 48 | Size_127_For_2_T | 0.005 | 236 | Size_148_For_5_G | 0.000 |

(continued)

| Importance by feature | | | | | |
|---|---|---|---|---|---|
| Rank | Feature | Importance | Rank | Feature | Importance |
| 49 | Size_158_For_2_T | 0.005 | 237 | Size_148_For_5_C | 0.000 |
| 50 | Size_156_For_3_G | 0.005 | 238 | Size_148_For_6_A | 0.000 |
| 51 | Size_148_For_2_C | 0.004 | 239 | Size_148_For_7_A | 0.000 |
| 52 | Size_137_For_2_C | 0.004 | 240 | Size_148_For_8_A | 0.000 |
| 53 | Size_127_For_1_G | 0.004 | 241 | Size_148_For_9_A | 0.000 |
| 54 | Size_181_For_1_A | 0.004 | 242 | Size_148_For_10_A | 0.000 |
| 55 | Size_129_For_3_T | 0.004 | 243 | Size_149_For_1_A | 0.000 |
| 56 | Size_150_For_1_G | 0.004 | 244 | Size_149_For_1_C | 0.000 |
| 57 | Size_127_For_2_A | 0.004 | 245 | Size_149_For_2_A | 0.000 |
| 58 | Size_137_For_3_G | 0.003 | 246 | Size_149_For_2_T | 0.000 |
| 59 | Size_158_For_2_A | 0.003 | 247 | Size_149_For_2_G | 0.000 |
| 60 | Size_157_For_1_C | 0.003 | 248 | Size_149_For_2_C | 0.000 |
| 61 | Size_181_For_2_T | 0.003 | 249 | Size_149_For_3_A | 0.000 |
| 62 | Size_148_For_4_G | 0.003 | 250 | Size_149_For_3_T | 0.000 |
| 63 | Size_182_For_2_C | 0.003 | 251 | Size_149_For_3_G | 0.000 |
| 64 | Size_149_For_1_T | 0.002 | 252 | Size_149_For_3_C | 0.000 |
| 65 | Size_150_For_5_T | 0.002 | 253 | Size_149_For_4_A | 0.000 |
| 66 | Size_157_For_3_G | 0.002 | 254 | Size_149_For_4_T | 0.000 |
| 67 | Size_127_For_3_C | 0.002 | 255 | Size_149_For_4_G | 0.000 |
| 68 | Size_183_For_1_A | 0.002 | 256 | Size_149_For_4_C | 0.000 |
| 69 | Size_156_For_5_T | 0.002 | 257 | Size_149_For_5_A | 0.000 |
| 70 | Size_139_For_3_C | 0.002 | 258 | Size_149_For_5_T | 0.000 |
| 71 | Size_183_For_1_C | 0.002 | 259 | Size_149_For_5_C | 0.000 |
| 72 | Size_138_For_2_A | 0.002 | 260 | Size_149_For_6_A | 0.000 |
| 73 | Size_158_For_3_A | 0.002 | 261 | Size_149_For_7_A | 0.000 |
| 74 | Size_157_For_1_T | 0.002 | 262 | Size_149_For_8_A | 0.000 |
| 75 | Size_150_For_3_T | 0.002 | 263 | Size_149_For_9_A | 0.000 |
| 76 | Size_128_For_3_G | 0.002 | 264 | Size_149_For_10_A | 0.000 |
| 77 | Size_158_For_3_G | 0.002 | 265 | Size_150_For_1_A | 0.000 |
| 78 | Size_127_For_3_T | 0.002 | 266 | Size_150_For_1_C | 0.000 |
| 79 | Size_127_For_5_C | 0.001 | 267 | Size_150_For_2_A | 0.000 |
| 80 | Size_182_For_1_G | 0.001 | 268 | Size_150_For_2_T | 0.000 |
| 81 | Size_156_For_2_A | 0.001 | 269 | Size_150_For_2_G | 0.000 |
| 82 | Size_158_For_4_T | 0.001 | 270 | Size_150_For_3_G | 0.000 |
| 83 | Size_137_For_5_A | 0.001 | 271 | Size_150_For_3_C | 0.000 |
| 84 | Size_183_For_1_G | 0.001 | 272 | Size_150_For_4_A | 0.000 |
| 85 | Size_137_For_1_C | 0.001 | 273 | Size_150_For_4_T | 0.000 |

(continued)

| Importance by feature | | | | | |
|---|---|---|---|---|---|
| Rank | Feature | Importance | Rank | Feature | Importance |
| 86 | Size_156_For_4_A | 0.001 | 274 | Size_150_For_4_G | 0.000 |
| 87 | Size_156_For_3_C | 0.001 | 275 | Size_150_For_4_C | 0.000 |
| 88 | Size_182_For_2_A | 0.001 | 276 | Size_150_For_5_A | 0.000 |
| 89 | Size_183_For_2_C | 0.001 | 277 | Size_150_For_5_G | 0.000 |
| 90 | Size_127_For_4_G | 0.001 | 278 | Size_150_For_5_C | 0.000 |
| 91 | Size_137_For_2_A | 0.001 | 279 | Size_150_For_6_A | 0.000 |
| 92 | Size_127_For_4_C | 0.001 | 280 | Size_150_For_7_A | 0.000 |
| 93 | Size_181_For_3_C | 0.001 | 281 | Size_150_For_8_A | 0.000 |
| 94 | Size_129_For_2_T | 0.001 | 282 | Size_150_For_10_A | 0.000 |
| 95 | Size_157_For_5_G | 0.001 | 283 | Size_156_For_1_A | 0.000 |
| 96 | Size_127_For_1_T | 0.001 | 284 | Size_156_For_2_G | 0.000 |
| 97 | Size_150_For_3_A | 0.001 | 285 | Size_156_For_2_C | 0.000 |
| 98 | Size_127_For_4_T | 0.001 | 286 | Size_156_For_3_A | 0.000 |
| 99 | Size_156_For_7_A | 0.001 | 287 | Size_156_For_4_T | 0.000 |
| 100 | Size_182_For_1_C | 0.001 | 288 | Size_156_For_5_A | 0.000 |
| 101 | Size_181_For_4_G | 0.001 | 289 | Size_156_For_5_C | 0.000 |
| 102 | Size_150_For_1_T | 0.001 | 290 | Size_156_For_6_A | 0.000 |
| 103 | Size_127_For_2_G | 0.000 | 291 | Size_156_For_8_A | 0.000 |
| 104 | Size_127_For_3_A | 0.000 | 292 | Size_156_For_9_A | 0.000 |
| 105 | Size_127_For_4_A | 0.000 | 293 | Size_156_For_10_A | 0.000 |
| 106 | Size_127_For_5_A | 0.000 | 294 | Size_157_For_1_G | 0.000 |
| 107 | Size_127_For_5_T | 0.000 | 295 | Size_157_For_2_G | 0.000 |
| 108 | Size_127_For_6_A | 0.000 | 296 | Size_157_For_2_C | 0.000 |
| 109 | Size_127_For_7_A | 0.000 | 297 | Size_157_For_3_C | 0.000 |
| 110 | Size_127_For_8_A | 0.000 | 298 | Size_157_For_4_A | 0.000 |
| 111 | Size_127_For_9_A | 0.000 | 299 | Size_157_For_4_T | 0.000 |
| 112 | Size_127_For_10_A | 0.000 | 300 | Size_157_For_4_G | 0.000 |
| 113 | Size_128_For_1_T | 0.000 | 301 | Size_157_For_4_C | 0.000 |
| 114 | Size_128_For_1_G | 0.000 | 302 | Size_157_For_5_A | 0.000 |
| 115 | Size_128_For_1_C | 0.000 | 303 | Size_157_For_5_T | 0.000 |
| 116 | Size_128_For_2_A | 0.000 | 304 | Size_157_For_5_C | 0.000 |
| 117 | Size_128_For_2_T | 0.000 | 305 | Size_157_For_6_A | 0.000 |
| 118 | Size_128_For_2_G | 0.000 | 306 | Size_157_For_7_A | 0.000 |
| 119 | Size_128_For_2_C | 0.000 | 307 | Size_157_For_8_A | 0.000 |
| 120 | Size_128_For_3_A | 0.000 | 308 | Size_157_For_9_A | 0.000 |
| 121 | Size_128_For_3_T | 0.000 | 309 | Size_157_For_10_A | 0.000 |
| 122 | Size_128_For_3_C | 0.000 | 310 | Size_158_For_3_C | 0.000 |

EP 4 428 864 A1

(continued)

| Importance by feature | | | | | |
|---|---|---|---|---|---|
| Rank | Feature | Importance | Rank | Feature | Importance |
| 123 | Size_128_For_4_A | 0.000 | 311 | Size_158_For_4_A | 0.000 |
| 124 | Size_128_For_4_T | 0.000 | 312 | Size_158_For_4_G | 0.000 |
| 125 | Size_128_For_4_G | 0.000 | 313 | Size_158_For_4_C | 0.000 |
| 126 | Size_128_For_4_C | 0.000 | 314 | Size_158_For_5_A | 0.000 |
| 127 | Size_128_For_5_A | 0.000 | 315 | Size_158_For_5_T | 0.000 |
| 128 | Size_128_For_5_T | 0.000 | 316 | Size_158_For_5_G | 0.000 |
| 129 | Size_128_For_5_G | 0.000 | 317 | Size_158_For_5_C | 0.000 |
| 130 | Size_128_For_5_C | 0.000 | 318 | Size_158_For_6_A | 0.000 |
| 131 | Size_128_For_6_A | 0.000 | 319 | Size_158_For_7_A | 0.000 |
| 132 | Size_128_For_7_A | 0.000 | 320 | Size_158_For_8_A | 0.000 |
| 133 | Size_128_For_8_A | 0.000 | 321 | Size_158_For_9_A | 0.000 |
| 134 | Size_128_For_9_A | 0.000 | 322 | Size_158_For_10_A | 0.000 |
| 135 | Size_128_For_10_A | 0.000 | 323 | Size_181_For_1_T | 0.000 |
| 136 | Size_129_For_1_A | 0.000 | 324 | Size_181_For_1_C | 0.000 |
| 137 | Size_129_For_1_T | 0.000 | 325 | Size_181_For_2_A | 0.000 |
| 138 | Size_129_For_1_G | 0.000 | 326 | Size_181_For_2_G | 0.000 |
| 139 | Size_129_For_1_C | 0.000 | 327 | Size_181_For_2_C | 0.000 |
| 140 | Size_129_For_2_A | 0.000 | 328 | Size_181_For_3_A | 0.000 |
| 141 | Size_129_For_2_G | 0.000 | 329 | Size_1 81_For_3_T | 0.000 |
| 142 | Size_129_For_2_C | 0.000 | 330 | Size_181_For_4_A | 0.000 |
| 143 | Size_129_For_3_A | 0.000 | 331 | Size_1 81_For_4_T | 0.000 |
| 144 | Size_129_For_3_G | 0.000 | 332 | Size_181_For_4_C | 0.000 |
| 145 | Size_129_For_3_C | 0.000 | 333 | Size_181_For_5_A | 0.000 |
| 146 | Size_129_For_4_A | 0.000 | 334 | Size_1 81_For_5_G | 0.000 |
| 147 | Size_129_For_4_T | 0.000 | 335 | Size_1 81_For_5_C | 0.000 |
| 148 | Size_129_For_4_G | 0.000 | 336 | Size_181_For_6_A | 0.000 |
| 149 | Size_129_For_4_C | 0.000 | 337 | Size_181_For_7_A | 0.000 |
| 150 | Size_129_For_5_A | 0.000 | 338 | Size_181_For_8_A | 0.000 |
| 151 | Size_129_For_5_T | 0.000 | 339 | Size_181_For_9_A | 0.000 |
| 152 | Size_129_For_5_G | 0.000 | 340 | Size_181_For_10_A | 0.000 |
| 153 | Size_129_For_5_C | 0.000 | 341 | Size_182_For_1_T | 0.000 |
| 154 | Size_129_For_6_A | 0.000 | 342 | Size_182_For_2_T | 0.000 |
| 155 | Size_129_For_7_A | 0.000 | 343 | Size_182_For_2_G | 0.000 |
| 156 | Size_129_For_8_A | 0.000 | 344 | Size_182_For_3_A | 0.000 |
| 157 | Size_129_For_9_A | 0.000 | 345 | Size_182_For_3_T | 0.000 |
| 158 | Size_129_For_10_A | 0.000 | 346 | Size_182_For_3_G | 0.000 |
| 159 | Size_137_For_1_T | 0.000 | 347 | Size_182_For_3_C | 0.000 |

20

(continued)

| Importance by feature | | | | | |
| Rank | Feature | Importance | Rank | Feature | Importance |
| --- | --- | --- | --- | --- | --- |
| 160 | Size_137_For_1_G | 0.000 | 348 | Size_182_For_4_A | 0.000 |
| 161 | Size_137_For_2_G | 0.000 | 349 | Size_182_For_4_G | 0.000 |
| 162 | Size_137_For_3_A | 0.000 | 350 | Size_182_For_4_C | 0.000 |
| 163 | Size_137_For_3_T | 0.000 | 351 | Size_182_For_5_A | 0.000 |
| 164 | Size_137_For_3_C | 0.000 | 352 | Size_182_For_5_G | 0.000 |
| 165 | Size_137_For_4_A | 0.000 | 353 | Size_182_For_6_A | 0.000 |
| 166 | Size_137_For_4_T | 0.000 | 354 | Size_182_For_7_A | 0.000 |
| 167 | Size_137_For_4_G | 0.000 | 355 | Size_182_For_9_A | 0.000 |
| 168 | Size_137_For_4_C | 0.000 | 356 | Size_182_For_10_A | 0.000 |
| 169 | Size_137_For_5_T | 0.000 | 357 | Size_183_For_2_A | 0.000 |
| 170 | Size_137_For_5_G | 0.000 | 358 | Size_183_For_2_T | 0.000 |
| 171 | Size_137_For_5_C | 0.000 | 359 | Size_183_For_2_G | 0.000 |
| 172 | Size_137_For_6_A | 0.000 | 360 | Size_183_For_3_A | 0.000 |
| 173 | Size_137_For_8_A | 0.000 | 361 | Size_183_For_3_T | 0.000 |
| 174 | Size_137_For_9_A | 0.000 | 362 | Size_183_For_3_C | 0.000 |
| 175 | Size_137_For_10_A | 0.000 | 363 | Size_183_For_4_A | 0.000 |
| 176 | Size_138_For_1_A | 0.000 | 364 | Size_183_For_4_T | 0.000 |
| 177 | Size_138_For_1_T | 0.000 | 365 | Size_183_For_4_G | 0.000 |
| 178 | Size_138_For_1_C | 0.000 | 366 | Size_183_For_4_C | 0.000 |
| 179 | Size_138_For_2_T | 0.000 | 367 | Size_183_For_5_A | 0.000 |
| 180 | Size_138_For_2_G | 0.000 | 368 | Size_183_For_5_T | 0.000 |
| 181 | Size_138_For_2_C | 0.000 | 369 | Size_183_For_5_G | 0.000 |
| 182 | Size_138_For_3_A | 0.000 | 370 | Size_183_For_5_C | 0.000 |
| 183 | Size_138_For_3_T | 0.000 | 371 | Size_183_For_6_A | 0.000 |
| 184 | Size_138_For_3_C | 0.000 | 372 | Size_183_For_7_A | 0.000 |
| 185 | Size_138_For_4_A | 0.000 | 373 | Size_183_For_8_A | 0.000 |
| 186 | Size_138_For_4_T | 0.000 | 374 | Size_183_For_9_A | 0.000 |
| 187 | Size_138_For_4_G | 0.000 | 375 | Size_183_For_10_A | 0.000 |
| 188 | Size_138_For_4_C | 0.000 | | | |

5-2. Checking TopN feature performance

As a result of checking the performance of the XGB model built using the method of Example 3 using only the top 1 feature, the model using up to 2 features, the model using up to 3, 4, 5, 6, 7, 8, 9, 15, 20, 25, 30, 35, 40, 45, and 50 features, it was found that the performance of the XGB model built using the method of Example 4 using top 5 features was sufficient as shown in Table 7 and FIG. 10.

[Table 7]

| Type | Top1 | Top2 | Top3 | Top4 | Top5 | Top6 | Top7 | Top8 | Top9 | Top10 | Top15 | Top20 | Top25 | Top30 | Top35 | Top40 | Top45 | Top50 | ALL |
|---|---|---|---|---|---|---|---|---|---|---|---|---|---|---|---|---|---|---|---|
| ACC_Train | 0.845 | 0.938 | 0.977 | 0.930 | 0.915 | 0.977 | 0.868 | 0.915 | 0.891 | 0.946 | 0.891 | 0.891 | 0.930 | 0.922 | 1.000 | 0.938 | 0.953 | 1.000 | 1.000 |
| ACC_Valid | 0.855 | 0.927 | 0.909 | 0.927 | 0.945 | 0.927 | 0.909 | 0.927 | 0.909 | 0.909 | 0.909 | 0.909 | 0.927 | 0.927 | 0.945 | 0.927 | 0.927 | 0.945 | 0.945 |
| ACC_Test | 0.911 | 0.873 | 0.899 | 0.924 | 0.987 | 0.949 | 0.886 | 0.937 | 0.886 | 0.911 | 0.886 | 0.886 | 0.861 | 0.873 | 0.911 | 0.899 | 0.886 | 0.937 | 0.937 |
| AUC_Train | 0.841 | 0.991 | 0.997 | 0.990 | 0.988 | 0.996 | 0.914 | 0.980 | 0.931 | 0.991 | 0.906 | 0.906 | 0.957 | 0.940 | 1.000 | 0.986 | 0.993 | 1.000 | 1.000 |
| AUC_Valid | 0.853 | 0.870 | 0.888 | 0.934 | 0.916 | 0.912 | 0.884 | 0.883 | 0.876 | 0.919 | 0.874 | 0.874 | 0.927 | 0.910 | 0.934 | 0.936 | 0.912 | 0.929 | 0.952 |
| AUC_Test | 0.963 | 0.926 | 0.931 | 0.965 | 0.979 | 0.971 | 0.964 | 0.974 | 0.952 | 0.933 | 0.938 | 0.938 | 0.919 | 0.933 | 0.974 | 0.948 | 0.945 | 0.979 | 0.987 |

In other words, the top three rows of Table 7 show the results of the Accuracy (ACC) method, and the bottom three rows show the results of the AUC method. The composition of the train, valid, and test sets for ACC and AUC performance is the same. Accuracy (ACC) is a performance indicator that measures whether the probability value predicted by the model is higher or lower than a set cutoff value (cutoff = 0.5), and AUC is a performance indicator that measures how clearly the distribution of predicted probability values differs between the normal group and the cancer patient group without setting a specific cutoff, unlike ACC.

For ACC, it makes sense to interpret the results based on the AUC value because the results can vary depending on how the cutoff value is set. Based on the AUC value of the test set, the results in Table 7 show that:

i) AUC is 0.987 when using all 375 features, which is the highest performance when compared to using some subset of features.

ii) Finding the smallest number of features to achieve similar Test AUC performance as when using 375 features, TopN is 5.

While the foregoing has described in detail certain aspects of the present invention, it would be apparent to one of ordinary skill in the art that these specific descriptions are merely preferred embodiments and are not intended to limit the scope of the present invention. Accordingly, the substantial scope of the present invention is defined by the appended claims and their equivalents.

Industrial Availability

The cancer diagnosis method using sequence relative frequency and size for each position of a cell-free nucleic acid fragment according to the present invention is useful because it obtains optimal sequence relative frequency and size information for each position of nucleic acid fragment and analyzes it using an AI algorithm, showing high sensitivity and accuracy even if read coverage is low.

**Claims**

1. A method of providing information for diagnosing cancer using a cell-free nucleic acid, comprising the following steps:

    (a) obtaining a sequence information from extracted nucleic acid from a biological sample;
    (b) aligning the obtained sequence information (reads) to a reference genome database;
    (c) deriving the sequence relative frequency and the size at each position of nucleic acid fragments using the aligned sequence information (reads); and
    (d) inputting the derived sequence relative frequency and size information to an artificial intelligence model trained to diagnose cancer and comparing the analyzed output with a cut-off value to determine the presence or absence of cancer,

    wherein the artificial intelligence model is trained to distinguish normal samples and cancer samples based on the sequence relative frequency at each position of the nucleic acid fragment and the size information of the nucleic acid fragment.

2. A method of diagnosing cancer using a cell-free nucleic acid comprising the following steps:

    (a) obtaining a sequence information from extracted nucleic acid from a biological sample;
    (b) aligning the obtained sequence information (reads) to a reference genome database;
    (c) deriving the sequence relative frequency and the size at each position of nucleic acid fragments using the aligned sequence information (reads); and
    (d) inputting the derived sequence relative frequency and size information to an artificial intelligence model trained to diagnose cancer and comparing the analyzed output with a cut-off value to determine the presence or absence of cancer,

    wherein the artificial intelligence model is trained to distinguish normal samples and cancer samples based on the sequence relative frequency at each position of the nucleic acid fragment and the size information of the nucleic acid fragment.

3. The method according to claim 1 or 2, wherein step (a) is performed by a method comprising the steps of:

    (a-i) obtaining nucleic acids from a biospecimen;
    (a-ii) removing proteins, fats, and other residues from the collected nucleic acid by using a salting-out method, a column chromatography method, or a beads method to obtain purified nucleic acid;
    (a-iii) creating a single-end sequencing or pair-end sequencing library from purified nucleic acids or nucleic acids that have been randomly fragmented by enzymatic cleavage, grinding, or hydroshear methods;
    (a-iv) reacting the created library to a next-generation sequencer; and
    (a-v) acquiring nucleic acid sequence information (reads) from a next-generation sequencer.

4. The method according to claim 1, wherein the size of the nucleic acid fragment in step (c) is selected from the group consisting of 127 to 129 bp, 137 to 139 bp, 148 to 150 bp, 156 to 158 bp, and 181 to 183 bp.

5. The method according to claim 1, wherein the sequence relative frequency for each position of the nucleic acid fragments in step (c) is the number of nucleic acid fragments having A, T, G and C bases detected at each position, normalized to the total number of nucleic acid fragments, in nucleic acid fragments of the same size.

6. The method according to claim 5, wherein the position of the nucleic acid fragment in step (c) is 1 to 10 bases at the 5' end of the nucleic acid fragment.

7. The method according to claim 5, the sequence relative frequency for each position of the nucleic acid fragments in step (c) may be the frequency of A, T, G and C bases at positions 1 to 5, and the frequency of A base at positions 6 to 10 at the 5' end of the nucleic acid fragment.

8. The method according to claim 1, wherein the sequence relative frequency of the nucleic acid fragments for each position and the size of the nucleic acid fragments in step (c) are at least one selected from those listed in Table 3.

9. The method according to claim 1, wherein the artificial intelligence model of step (d) is selected from the group consisting of AdaBoost, Random forest, Catboost, Light Gradient Boosting Model, and XGBoost.

10. The method according to claim 9, wherein the artificial intelligence model is XGBoost, and when learning binary classification, the loss function is represented by Formula 1 below:

Formula 1:

$$\text{loss}(\text{model}(x), y) = -\frac{1}{n}\left[\sum_{i=1}^{n}\left(y_i \log\big(model(x_i)\big) + (1 - y_i)\log\big(1 - model(x_i)\big)\right)\right]$$

model($x_i$) = *Output of the XGboost model for the i_th input*
y = Actual label value
n = Number of input data

11. The method according to claim 1, wherein the resultant value of the analysis from the sequence relative frequency and size information with the artificial intelligence model input in step d) is a XPI (XGBoost Probability Index) value.

12. The method according to claim 1, wherein the cut-off value of step (d) is 0.5, and wherein a value greater than 0.5 determines that the subject has cancer.

13. A cancer diagnostic device comprising:

   a decoding part that extracts nucleic acid from a biospecimen and decodes sequence information;
   an alignment part that aligns the decoded sequence to a reference genome database;
   a nucleic acid fragment analysis part that derives the sequence relative frequency at each position of the nucleic acid fragment and the size of the nucleic acid fragment based on the aligned sequence; and
   a cancer diagnostic part that inputs the derived sequence relative frequency at each position of the nucleic acid fragment and size information of the nucleic acid fragment into a trained artificial intelligence model, analyzes same, and determines the presence of cancer by comparing the analyzed output to a cut-off value.

14. A computer-readable storage medium comprising instructions configured to be executed by a processor to provide information for diagnosing cancer, the instructions comprising:

   (a) extracting a nucleic acid from a biospecimen to obtain sequence information;
   (b) aligning the obtained sequence information (reads) to a reference genome database;
   (c) deriving the sequence relative frequency and the size at each position of nucleic acid fragments using the aligned sequence information (reads); and
   (d) inputting the derived sequence relative frequency and size information to an artificial intelligence model trained to diagnose cancer and comparing the analyzed output with a cut-off value to determine the presence or absence of cancer, wherein the artificial intelligence model is trained to distinguish normal samples and cancer samples based on the sequence relative frequency at each position of the nucleic acid fragment and the size information of the nucleic acid fragment.

**FIG. 1**

```
┌─────────────────────────────────────────────────────────┐
│      Extracting cell-free nucleic acids from plasma      │
└─────────────────────────────────────────────────────────┘
                            │
                            ▼
┌─────────────────────────────────────────────────────────┐
│  Acquiring nucleic acid fragments by using massively     │
│                parallel sequencing method                │
└─────────────────────────────────────────────────────────┘
                            │
                            ▼
┌─────────────────────────────────────────────────────────┐
│   Aligning nucleic acid fragment data human reference    │
│  chromosomes and Determining quality of aligned data     │
└─────────────────────────────────────────────────────────┘
                            │
                            ▼
┌─────────────────────────────────────────────────────────┐
│     Measuring length of aligned nucleic acid fragments   │
└─────────────────────────────────────────────────────────┘
                            │
                            ▼
┌─────────────────────────────────────────────────────────┐
│ Calculating nucleic acid sequence frequency at each      │
│ position of nucleic acid fragment in nucleic acid        │
│ fragment group with same size                            │
└─────────────────────────────────────────────────────────┘
                            │
                            ▼
┌─────────────────────────────────────────────────────────┐
│ Screening the optimal combinations of nucleic acid       │
│ fragment size, position and base sequence to distinguish │
│ between healthy individuals and cancer patients          │
└─────────────────────────────────────────────────────────┘
                            │
                            ▼
┌─────────────────────────────────────────────────────────┐
│              Training machine learning model             │
└─────────────────────────────────────────────────────────┘
                            │
                            ▼
┌─────────────────────────────────────────────────────────┐
│              Calculating probability index               │
└─────────────────────────────────────────────────────────┘
                            │
                            ▼
┌─────────────────────────────────────────────────────────┐
│    Determining whether the subject is a cancer patient   │
└─────────────────────────────────────────────────────────┘
```

FIG. 2

## FIG. 3

Training Dataset

Validation Dataset

**FIG. 4**

**FIG. 5**

**FIG. 6**

FIG. 7

**(A)**

**(B)**

FIG. 8

Train   Valid   Test

FIG. 9

Train_For_1 / A

Train_Rev_59 / G

FIG. 10

**Train**

**Valid**

**Test**

**Train**

**Valid**

**Test**

| INTERNATIONAL SEARCH REPORT | International application No. |
|---|---|
| | **PCT/KR2022/016868** |

**A. CLASSIFICATION OF SUBJECT MATTER**

**G16B 35/00**(2019.01)i; **G16B 30/10**(2019.01)i; **G16B 5/00**(2019.01)i; **G16B 40/20**(2019.01)i; **C12Q 1/6886**(2018.01)i; **C12Q 1/6806**(2018.01)i

According to International Patent Classification (IPC) or to both national classification and IPC

**B. FIELDS SEARCHED**

Minimum documentation searched (classification system followed by classification symbols)

G16B 35/00(2019.01); G16B 20/00(2019.01); G16B 30/00(2019.01); G16B 40/00(2019.01); G16B 50/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 무세포 핵산(cell free nucleic acid), 핵산단편(fragments), 상대 빈도(relative frequency), 크기(volume), 인공지능(artificial intelligence), 암(cancer), 진단(diagnosis)

**C. DOCUMENTS CONSIDERED TO BE RELEVANT**

| Category* | Citation of document, with indication, where appropriate, of the relevant passages | Relevant to claim No. |
|---|---|---|
| A | CRISTIANO, Stephen et al. Genome-wide cell-free DNA fragmentation in patients with cancer. Nature. 29 May 2019 (online publication date), vol. 570, pp. 385-389, METHODS.<br>See pages 385-389, and METHODS. | 1-14 |
| DA | KR 10-2021-0113237 A (THE CHINESE UNIVERSITY OF HONG KONG et al.) 15 September 2021 (2021-09-15)<br>See paragraphs [0018], [0034]-[0036], [0042]-[0052], [0106] and [0107]; and figures 13 and 14. | 1-14 |
| A | WAN, Nathan et al. Machine learning enables detection of early-stage colorectal cancer by whole-genome sequencing of plasma cell-free DNA. BMC Cancer. 23 August 2019 (online publication date), vol. 19, article no. 832, inner pp. 1-10.<br>See inner pages 2-6. | 1-14 |
| A | KR 10-2019-0085667 A (GREEN CROSS GENOME CORPORATION) 19 July 2019 (2019-07-19)<br>See paragraphs [0024], [0031]-[0038] and [0040]-[0046]. | 1-14 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 February 2023** | **10 February 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/016868**

**C.    DOCUMENTS CONSIDERED TO BE RELEVANT**

| Category* | Citation of document, with indication, where appropriate, of the relevant passages | Relevant to claim No. |
|---|---|---|
| A | KR 10-2019-0036494 A (EWHA UNIVERSITY - INDUSTRY COLLABORATION FOUNDATION) 04 April 2019 (2019-04-04)<br>See paragraphs [0023], [0027], [0028], [0032], [0033] and [0037]. | 1-14 |

Form PCT/ISA/210 (second sheet) (July 2022)

**INTERNATIONAL SEARCH REPORT**
Information on patent family members

International application No.

**PCT/KR2022/016868**

| Patent document cited in search report | | | Publication date (day/month/year) | Patent family member(s) | | | Publication date (day/month/year) |
|---|---|---|---|---|---|---|---|
| KR | 10-2021-0113237 | A | 15 September 2021 | AU | 2019-410635 | A1 | 17 June 2021 |
| | | | | CA | 3123474 | A1 | 25 June 2020 |
| | | | | CN | 113366122 | A | 07 September 2021 |
| | | | | EP | 3899018 | A1 | 27 October 2021 |
| | | | | JP | 2022-514879 | A | 16 February 2022 |
| | | | | US | 2020-0199656 | A1 | 25 June 2020 |
| | | | | WO | 2020-125709 | A1 | 25 June 2020 |
| KR | 10-2019-0085667 | A | 19 July 2019 | KR | 10-2029393 | B1 | 07 October 2019 |
| | | | | WO | 2019-139363 | A1 | 18 July 2019 |
| KR | 10-2019-0036494 | A | 04 April 2019 | KR | 10-2233740 | B1 | 30 March 2021 |
| | | | | WO | 2019-066421 | A2 | 04 April 2019 |
| | | | | WO | 2019-066421 | A3 | 04 July 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10975431 B
- WO 2020125709 A
- KR 102061800
- KR 102108050
- KR 1020210081547

**Non-patent literature cited in the description**

- **ZHOU, XIONGHUI et al.** *bioRxiv*
- **PEIYONG JIANG et al.** *cancer discovery,* 2020, vol. 10, 664-673
- **DAPING YU et al.** *Thoracic Cancer,* 2020, vol. 11, 95-102
- **METZKER, M.** *Nature Biotechnology Reviews,* 2010, vol. 11, 31-46